# EUROPÄISCHE PATENTSCHRIFT

(11) **EP 0 549 645 B1**
(45) Veröffentlichungstag und Bekanntmachung des Hinweises auf die Patenterteilung: **31.08.1994**
(21) Anmeldenummer: 91916476.4
(22) Anmeldetag: 14.09.1991
(51) Int. Cl.: C07J 51/00, A61K 31/565

(54) **NEUE 17- -ÖSTRADIOLDERIVATE, VERFAHREN ZU DEREN HERSTELLUNG UND DIESE VERBINDUNGEN ENTHALTENDE ARZNEIMITTEL**
NEW 17- -OESTRADIOL DERIVATIVES, A METHOD OF PREPARING THEM, AND DRUGS CONTAINING THEM
NOUVEAUX DERIVES DE 17- -OESTRADIOL, LEUR PROCEDE DE PRODUCTION ET MEDICAMENTS CONTENANT CES COMPOSES

(30) Priorität: 18.09.1990 DE 4029499
(43) Veröffentlichungstag der Anmeldung: 07.07.1993
(73) Patentinhaber: BOEHRINGER MANNHEIM GMBH, 68298 Mannheim (DE)
(72) Erfinder: BOSIES, Elmar, D-6940 Weinheim (DE); ESSWEIN, Angelika, D-7700 Singen (DE); BAUSS, Frieder, D-6715 Lambsheim (DE)
(74) Vertreter: Weber, Manfred, Dr.
(86) Internationale Anmeldenummer: EP9101757
(87) Internationale Veröffentlichungsnummer: WO9205187

(56) Entgegenhaltungen:
- EP-A- 0 341 961
- AU-A- 457 784

## Beschreibung

Die vorliegende Erfindung betrifft neue 17-β-Östradiolderivate, Verfahren zu deren Herstellung sowie Arzneimittel, die diese Substanzen enthalten.

Zur Behandlung der postmenopausalen Osteoporose werden heutzutage neben Natriumfluorid bzw. Natriumfluorophosphat vor allem 17-β-Östradiol bzw. Derivate hiervon eingesetzt. (Med. Klinik 82, 238 (1987); Am. J. Med. 85, 847 (1988). 17-β-Östradiol bzw. Derivate hiervon wirken zum einen hemmend auf die Knochenresorption, zum anderen aber auch stimulierend auf die knochenaufbauenden Osteoblasten. Nachteilig wirkt sich jedoch aus, daß man nicht nur die gewünschte Wirkung am Knochen erhält, sondern daß diese Präparate auch eine systemische Wirkung zeigen. In diesem Zusammenhang wird die Gefahr eines erhöhten Brustkrebsrisiko diskutiert. Es stellt sich somit die Aufgabe, ein 17-β-Östradiolderivat zu finden, das keine systemische, sondern nur noch eine Wirkung am Knochen zeigt. Dieses Ziel kann man erreichen, indem man das 17-Östradiol an einen Carrier koppelt, der eine hohe Affinität zum Hydroxylapatit des Knochens zeigt. Ähnliche Konjugate sind z. B. in der EP 201.057 publiziert, wo Kopplungsprodukte zwischen Carbonanhydrase-Inhibitoren mit Tetracyclin bzw. Diphosphonaten beschrieben sind. In der EP 341.961 wird u.a. eine Verbindung beschrieben, bei der 17-β-Östradiol mit einer Tetracarbonsäure gekoppelt ist. Die vorliegende Erfindung beschreibt nun Verbindungen, bei denen 17-β-Östradiol über einen in-vivo hydrolisierbaren Spacer an ein 1,1-Diphosphonat gekoppelt ist. Diese Substanzen haften auf Grund des 1,1-Diphosphonatteils sehr gut am Knochen und werden dann durch Esterasen am Knochen in 17-β-Östradiol und eine Diphosphonsäure haltigen Rest gespalten, der auf Grund seiner besonderen chemischen Struktur aber nicht knochenresorptionshemmend wirkt.

Gegenstand der vorliegenden Erfindung sind demnach Verbindungen der allgemeinen Formel I 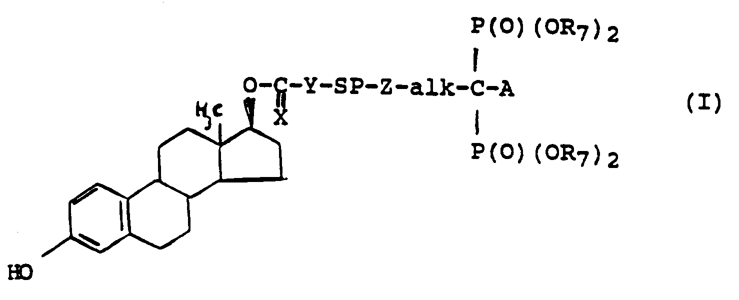
in der
- Sp: 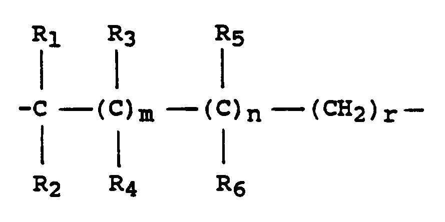
- R₁-R₆ =: jeweils unabhängig voneinander Wasserstoff, Phenyl, Cyclohexyl, niederes geradkettiges oder verzweigtes, Alkyl, Alkenyl oder Alkinyl mit 1-5 bzw. 2-5 Kohlenstoffatomen, das gegebenenfalls durch Phenyl substituiert ist, wobei R₁ und R₂ zusammen mit dem Kohlenstoffatom, an das sie gebunden sind einen gesättigten alicyclischen 3-6 Ring bilden können, bzw. R₁ und R₅ zusammen mit dem/den sie verbindenden Kohlenstoffatom(en) einen gegebenenfalls durch 1 oder 2 Stickstoffatome unterbrochenen gesättigten, teilgesättigten oder ungesättigten 3-6-Ring bilden können,
- m, n und r: unabhängig voneinander Werte von 0-3 einnehmen können, wobei m + n + r höchstens den Wert 7 einnehmen darf,
und
- X =: 0 oder S
- Y =: Valenzstrich oder NH
- Z =: Valenzstrich, 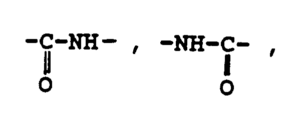 NH oder S,
- A =: Wasserstoff, Hydroxy oder gegebenenfalls durch niederes Alkyl substituiertes Amino,
- alk =: eine geradkettige oder verzweigte Alkylenkette mit 1-6 Kohlenstoffatomen,
- R₇ =: Wasserstoff oder niederes Alkyl
bedeuten, sowie deren pharmakologisch unbedenklichen Salze.

Niederes Alkyl bei den Resten R₁-R₇ bedeutet Alkylgruppen mit 1-5 Kohlenstoffatomen, vorzugsweise den Methyl-, Ethyl-, Propyl-, Isopropyl-, Isobutyl-, sec.-Butyl- und Pentylrest, insbesondere aber den Methyl-, Isopropyl, Isobutyl und sec.-Butylrest.

Bei dem Rest "A" hat niederes Alkyl bevorzugt die Bedeutung Methyl und Ethyl.

Alkenyl bei den Resten R₁-R₆ bedeutet Reste mit vorzugsweise einer Doppelbindung, vor allem den Allyl- und Methallylrest.

Alkinyl bei den Resten R₁-R₆ soll insbesondere der Propargylrest sein.

Unter der durch einen Phenylring substituierten Alkylgruppe versteht man insbesondere die Benzylgruppe.

Die Alkylenkette bei dem Rest "alk" soll Reste wie Methylen, Ethylen, Propylen, Isopropylen, Butylen, Isobutylen, Pentylen und Hexylen darstellen, insbesondere aber Methylen, Ethylen, Propylen, Butylen und Isobutylen.

Wenn R₁ und R₂ zusammen mit dem Kohlenstoffatom, an das sie gebunden sind, einen Ring bilden, soll dies vorzugsweise ein Fünf- bzw. Sechsring sein.

Für den Fall, daß R₁ und R₅ zusammen mit dem/den sie verbindenden Kohlenstoffatom(en) einen Ring bilden, ist dies ein Cyclopropyliden-, Cyclobutyliden-, Cyclopentyliden-, Cyclohexyliden-, Cyclohexenyliden- und Phenylenring, vorzugsweise der Cyclopropyliden, 1.2-Cyclopentyliden-, 1.2-Cyclohexyliden-, 1.3-Cyclohexyliden-, 1.4-Cycloheryliden-, 1.2-Cyclohex-4-enyliden-, 1.2-Phenyliden, 1.3-Phenyliden- und 1.4-Phenylidenring. Falls diese Ringe durch 1 oder 2 Stickstoffatome unterbrochen sind, handelt es sich vorzugsweise um ungesättigte Sechsringsysteme, insbesondere den in 2.3- bzw. 3.4-Stellung substituierten Pyridin- und den in 2.3-Stellung substituierten Pyrazinring.

Asymmetrische Kohlenstoffatome können die R- oder S-Konfiguration besitzen, und die Verbindungen können in optisch aktiver Form oder als racemisches Gemisch vorliegen. Sie sind ebenfalls Gegenstand der Erfindung.

Cis-trans-Isomere können als reine Formen oder als Gemische vorliegen und sind ebenfalls Gegenstand der Erfindung. Folgende Gruppen sind als "Spacer" (Sp) besonders bevorzugt: unsubstituiertes Methylen, durch Methyl, n-Pentyl, Allyl, iso-Butyl, tert.-Butyl, Cyclohexyl, Phenyl oder Benzyl monosubstituiertes Methylen, durch Methyl, Ethyl, n-Pentyl oder Allyl disubstituiertes Methylen, unsubstituiertes Ethylen, durch Methyl, n-Hexyl oder 1.1.-Cyclohexyliden monosubstituiertes Ethylen, durch Methyl disubstituiertes Ethylen, unsubstituiertes Propylen, durch Methyl, Phenyl, 1.1-Cyclopentyliden oder 1.1-Cyclohexyliden monosubstituiertes Propylen, durch Methyl disubstituiertes Propylen, unsubstituiertes Butylen, durch Methyl tetrasubstituiertes Butylen, Pentylen, Hexylen, Octylen, 1.1-Cyclopropyliden, 1.2-Cyclopropyliden, 1.1-Cyclobutyliden, 1.2-Cyclobutyliden, 1.1.-Cyclopentyliden, 1.2-Cyclopentyliden, 1.3-Cyclopentyliden, 1.1-Cyclohexyliden, 1.2-Cyclohexyliden, 1.3-Cyclohexyliden, 1.4-Cyclohexyliden, 1.2-Cyclo-hex-4-enyliden, 1.2-Phenylen, 1.3-Phenylen, 1.4-Phenylen, 4-Methylencyclohexyl, 4-Ethylidenphenyl, in 2.3-substituiertes Pyridin, in 3.4 substituiertes Pyridin und in 2.3 substituiertes Pyrazin.

Verbindungen der allgemeinen Formel I werden nach an sich bekannten Verfahren hergestellt, vorzugsweise indem man,
I für den Fall, daß Z die 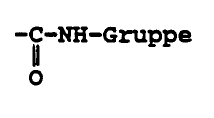 bedeutet,
   ein Carbonsäurederivat der allgemeinen Formel II 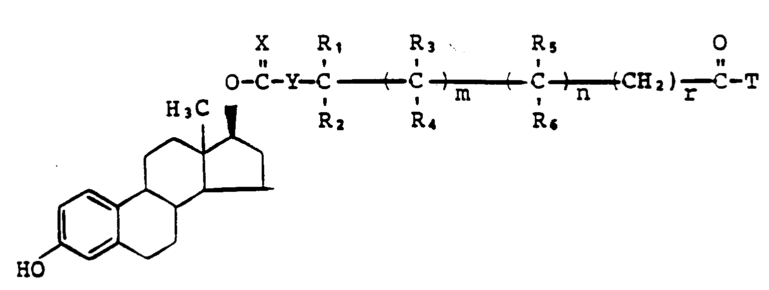 in der X, Y, R₁-R₆, m, n und r die oben angegebenen Bedeutungen haben und T die Hydroxigruppe oder eine Aktivierungsgruppe darstellt, mit einem Diphosphonsäurederivat der allgemeinen Formel III 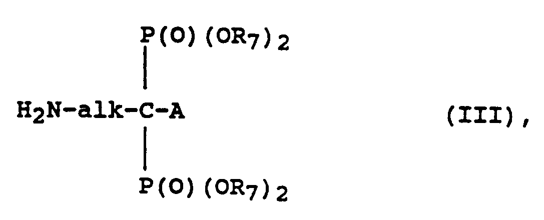 in der alk, R₇ und A die oben angegebenen Bedeutungen haben, umsetzt, oder
II) für den Fall, daß Z die Gruppe 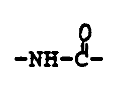 bedeutet, eine Aminoverbindung der allgemeinen Formel IV 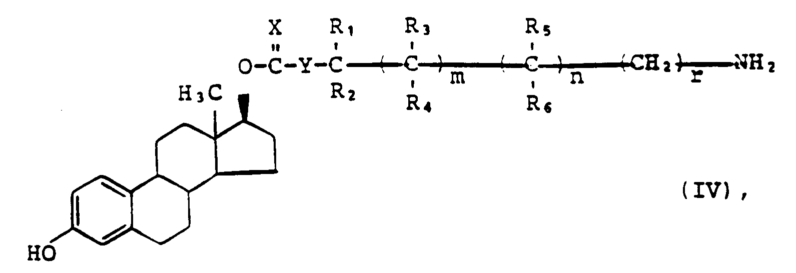 in der X, Y, R₁-R₆, m, n und r die oben genannten Bedeutungen haben, mit einem Diphosphonsäurederivat der allgemeinen Formel V 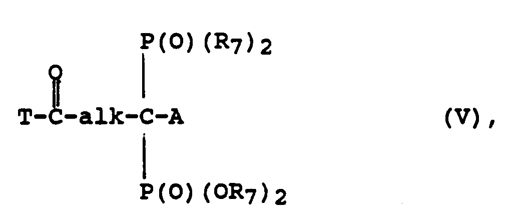 in der alk, A und R₇ die oben angegebenen Bedeutungen haben und T die Hydroxi- oder eine Aktivierungsgruppe darstellt, umsetzt, oder
III für den Fall, daß Z = NH oder S bedeutet, eine Verbindung der allgemeinen Formel VI 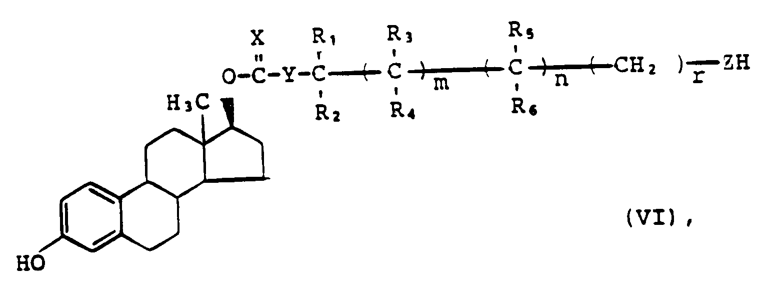 in der X, Y, R₁-R₆, m, n und r die oben genannten Bedeutungen haben und Z = NH oder S ist, mit einem Diphosphonsäurederivat der allgemeinen Formel VII 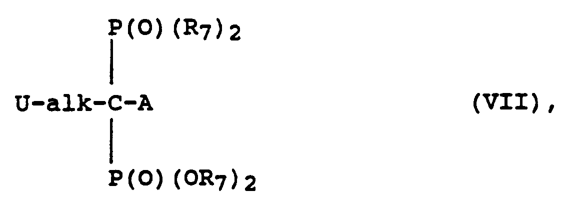 wobei alk, A und R₇ die oben angegebenen Bedeutungen haben und U einen reaktiven Rest darstellt, wobei für den Fall, daß alk eine C₁-Gruppe bedeutet, U und A zusammen auch einen Valenzstrich oder ein Sauerstoffatom darstellen können, umsetzt, oder
IV) für den Fall, daß X = Sauerstoff und Y = Valenzstrich darstellen, eine Verbindung der allgemeinen Formel VIII 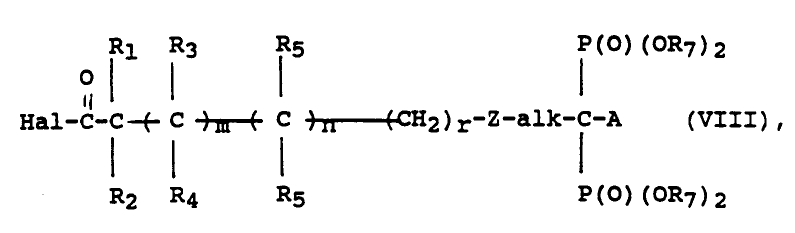 in der R₁-R₇, Z, alk, A, m, n und r die oben angegebenen Bedeutungen haben und Hal = Chlor oder Brom ist, mit einem 17-β-Östradiolderivat der allgemeinen Formel IX 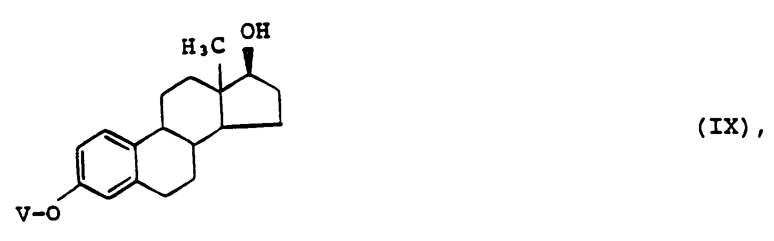 in der V eine Schutzgruppe darstellt, umsetzt, oder
V) für den Fall, daß Y = NH ist,
   a) eine Verbindung der allgemeinen Formel X 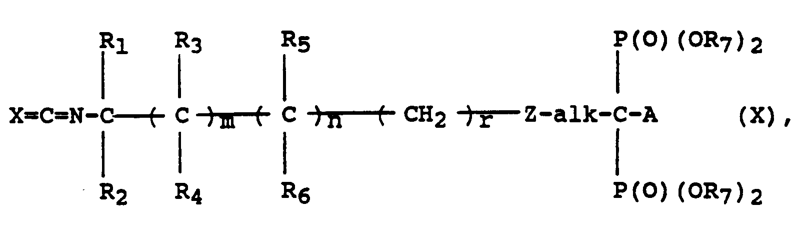 in der X, R₁-R₇, Z, alk, A, m, n und r die oben angegebenen Bedeutungen haben, mit einem 17-β-Östradiolderivat der allgemeinen Formel IX 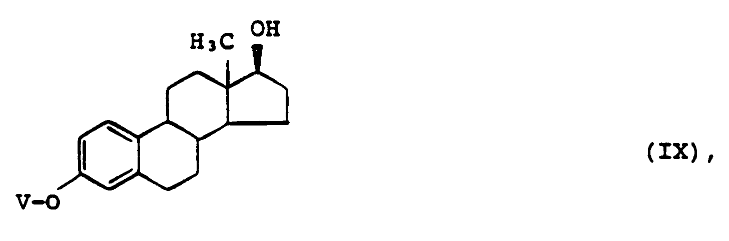 in der V eine Schutzgruppe darstellt, umsetzt, oder
   b) eine Aminoverbindung der allgemeinen Formel XI 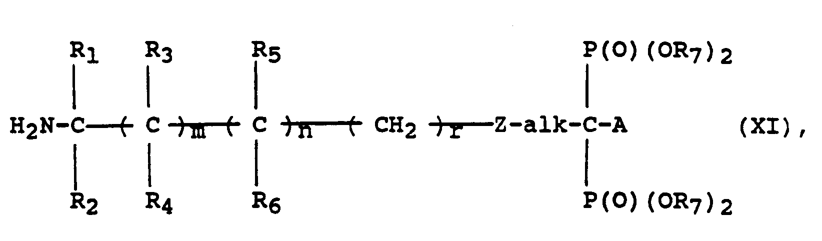 in der R₁-R₇, Z, alk, A, m, n und r die oben angegebenen Bedeutungen haben, mit einem 17-β-Östradiolderivat der allgemeinen Formel XII 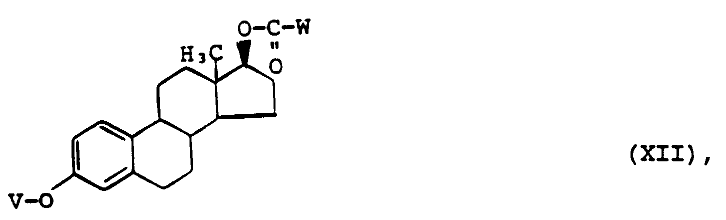 in der V eine Schutzgruppe darstellt und W = Chlor oder die Gruppe -X-M sein soll, wobei M niederes Alkyl wie Methyl oder Ethyl oder einen gegebenenfalls substituierten Phenylring darstellt und X die oben angegegebene Bedeutung hat, umsetzt, und anschließend gewünschtenfalls die C=O-Gruppe in eine C=S-Gruppe überführt oder
VI) Verbindungen der allgemeinen Formel I, in der X = Sauerstoff darstellt, nachträglich in Verbindungen der allgemeinen Formel I mit X = Schwefel umwandelt,
   die gegebenenfalls vorhandenen Schutzgruppen abspaltet und gewünschtenfalls die entstandenen Tetraester zu Diestern oder Säuren der allgemeinen Formel I verseift.

Die in Verfahren I und II angegebene Gruppierung 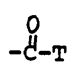
kann ein reaktives Derivat einer Carbonsäure darstellen. Hierfür kommen gemischte Anhydride, insbesondere mit Kohlensäureniederalkylestern wie Ethyl-, oder Isobutylestern oder aktive Ester, insbesondere p-Nitrophenyl-, 2.4.5-Trichlorphenyl-, N-Hydroxisuccinimid oder 1-Hydroxibenzotriazolester in Betracht. Stellt T eine Hydroxigruppe dar, kann die Aktivierung der Carboxylgruppe nach dem Carbodiimidverfahren durchgeführt werden. Es werden vorzugsweise inerte organische Lösungsmittel wie z.B. Methylenchlorid, Aceton, Acetonitril, Dimethylformamid, Tetrahydrofuran oder Dioxan oder Mischungen dieser Lösungsmittel bei Temperaturen von -70 bis +100°C, bevorzugt zwischen -30 und +20°C verwendet. Werden bei der Umsetzung die Phosphonsäuregruppen durch Salzbildung geschützt, so werden die Reaktionen vorzugsweise in wässrigem Medium, evtl. unter Zusatz von veretherten Diolen wie z.B. Dimethoxyethan oder in einem Zweiphasensystem wie z.B. Wasser/Methylenchlorid durchgeführt, wenn als reaktives Derivat der Verbindung der allgemeinen Formel II ein gemischtes Anhydrid oder ein Aktivester, vorzugsweise der N-Hydroxisuccinimidester verwendet wird.

Bei Verfahren III bedeutet der reaktive Rest U in Verbindungen der allgemeinen Formel VII einen Halogenrest, vorzugsweise das entsprechende Iodid oder Bromid oder ein Sulfonat wie z.B. das Mesylat, Benzolsulfonat oder p-Toluolsulfonat.

Falls "U" mit "A" zusammen einen Valenzstrich darstellen, handelt es sich bei der Verbindung der allgemeinen Formel VII um ein Methylenmethandiphosphonsäurederivat, falls "U" und "A" zusammen ein Sauerstoffatom bedeuten, handelt es sich um ein Oxiran-1.1-diphosphonsäurederivat. Die Umsetzungen werden in der Regel in inerten Lösungsmitteln wie Methylenchlorid, Dimethylformamid, Dioxan oder Tetrahydrofuran aber auch in Alkoholen wie Methanol oder Ethanol bei Temperaturen zwischen 0 und 100°C, vorzugsweise 0 und 30°C in Gegenwart von Basen wie Triethylamin, Lutidin oder Diazabicycloundecen durchgeführt. Man kann vor allem bei der Umsetzung von Thiolen der allgemeinen Formel VI auch sehr gut zuerst mit Hilfe von z.B. Alkalialkoholaten wie Natriummethylat oder -ethylat oder Kalium-tert.-butylat das Thiolat herstellen und dieses anschließend mit der Verbindung der allgemeinen Formel VII zur Reaktion bringen.

Die Umsetzung eines Säurehalogenids im Verfahren IV führt man üblicherweise in inerten Lösungsmitteln wie Methylenchlorid, Acetonitril, Dioxan, Tetrahydrofuran oder Dimethylformamid in Gegenwart von Basen wie Triethylamin, Lutidin oder auch festem Natriumhydrogencarbonat bei Temperaturen von -20 bis +30°C durch. Die Schutzgruppe V in der Verbindung der allgemeinen Formel IX bei Verfahren IV und V stellt entweder eine Acylgruppe wie z.B. die Formyl- oder Acetylgruppe oder die Benzyl- bzw. Tritylgruppe dar. Die Abspaltung der Acylgruppen geschieht vorzugsweise durch Umsetzung mit verdünntem wässrigen Ammoniak oder durch Behandlung mit Alkalialkoholaten wie Natriummethylat oder -ethylat. Die Benzyl- und Tritylgruppe kann durch katalytische Hydrierung in Gegenwart von Edelmetallkatalysatoren wie z.B. Palladium auf Kohle abgespalten werden.

Bei Verfahren V führt man die Reaktionen der beiden Verbindungen der allgemeinen Formel IX und X bzw. XI und XII in inerten Lösungsmitteln wie Methylenchlorid, Acetonitril, Dimethylformamid, Dioxan oder Tetrahydrofuran bei Temperaturen von 0 bis +40°C, in der Regel bei Zimmertemperatur durch. Falls "W" in der Verbindung der allgemeinen Formel XII Chlor bedeutet, setzt man noch Basen wie Triethylamin oder Lutidin zu.

Die nachträgliche Umwandlung bei Verfahren VI von Verbindungen der allgemeinen Formel I mit X = Sauerstoff in Verbindungen mit X = Schwefel erfolgt z.B. durch Umsetzung mit P₄S₁₀ (Liebigs Ann. 746, 92 (1971)) oder mit dem Lawesson's-Reagenz (Bull.Soc.Chim. Belg. 87, 223, 229, 525 (1978)).

Die bei Verfahren I eingesetzten Verbindungen der allgemeinen Formel II kann man für den Fall, daß Y = Valenzstrich ist, z.B. durch Umsetzung von 17-β-Östradiol ,mit einem Dicarbonsäureanhydrid herstellen (s. z.B. J. Biol. Chem. 253, 8221).

Für den Fall, daß Y in der Verbindung der allgemeinen Formel II = NH bedeutet, erhält man die gewünschten Verbindungen durch Reaktion von 17-β-Östradiol mit einer Isocyanato- oder Isothiocyanatocarbonsäure. Diese Substanzen sind literaturbeschrieben (z.B. Liebigs Ann. 575, 217, Liebigs Ann. 636, 144 oder J. Org. Chem. 28, 71) oder lassen sich analog den dort beschriebenen Verfahren herstellen.

Aminoalkyldiphosphonsäurederivate der allgemeinen Formel III lassen sich gut durch Reduktion der entsprechenden Nitrilverbindungen herstellen. Als Reduktionsverfahren wählt man vorzugsweise die katalytische Hydrierung in Gegenwart von Edelmetallkatalysatoren, insbesondere von Palladium auf Kohle, wobei in diesem Fall die Hydrierung vorzugsweise in einem Alkohol wie Methanol oder Ethanol in Gegenwart einer Halogenwasserstoffsäure wie z.B. Salzsäure vorgenommen wird. Die eingesetzten Nitrile lassen sich z.B. durch Reaktion eines entsprechenden Nitrilalkylhalogenids wie z.B. Brom- oder Chloracetonitril mit dem Alkalisalz eines Methandiphosphonsäuretetraalkylesters in Lösungsmitteln wie Toluol oder Dimethylformamid herstellen. Man erhält solche Verbindungen aber auch durch Addition eines gegebenenfalls substituierten Acrylnitrils an ein Alkalisalz eines Methandiphosphonsäuretetraalkylesters (s. EP 098 567). In diesen Fällen erhält man Verbindungen der allgemeinen Formel III mit A = Wasserstoff. Die Synthese von Aminoalkyl-diphosphonsäurederivaten, in denen A = Hydroxi bedeutet, ist im J. prakt. Chemie 321, 361 beschrieben.

Bei Verfahren II kann man die Aminoverbindungen der allgemeinen Formel IV für den Fall, daß Y einen Valenzstrich darstellt, durch Umsetzung eines am phenolischen Sauerstoff geschützten 17-β-Östradiols der allgemeinen Formel IX mit einer am Stickstoffatom geschützten Aminosäure, deren Carboxylgruppe aktiviert ist, erhalten (hierzu s. Beschreibung zu Verfahren V a). Die am phenolischen Sauerstoff eingesetzte Schutzgruppe ist vorzugsweise eine Benzyl- oder Tritylgruppe, die anschließend durch katalytische Hydrierung in Gegenwart von Edelmetallkatalysatoren wie z.B. Palladium auf Kohle abgespalten werden kann. Es kann als Schutzgruppe jedoch auch eine Acyl-, wie z.B. die Acetylgruppe verwendet werden (Tetrahedron Letters, 1979, 2431), die anschließend in schwach alkalischem Medium abgespalten werden kann.

Für den Fall, daß Y die NH-Gruppe bedeutet, kann man die entsprechenden am primären Stickstoffatom geschützten Aminoisocyanato- bzw. Aminoisothiocyanatoverbindungen mit einem am phenolischen Sauerstoffatom geschützten 17-β-Östradiol (s. oben) unter den bei Verfahren V a angeführten Bedingungen zur Reaktion bringen. Die hierzu benötigten, am primären Stickstoffatom geschützten Aminoisocyanato- bzw. Aminoisothiocyanatoverbindungen sind teilweise bekannt (J. Am. Chem. Soc. 72, 1620 bzw. 75, 3469; J. Org. Chem. 43, 1544; J. Ind. Chem. Soc. 47, 1143) oder lassen sich nach den dort angegebenen Methoden herstellen. Die Schutzgruppe stellt in diesem Fall vorzugsweise eine Acyl, z.B. eine Acetyl- oder Benzoylgruppe dar, die nach erfolgter Reaktion durch saure oder alkalische Hydrolyse wieder abgespalten wird.

Diphosphonsäurederivate der allgemeinen Formel V kann man auf folgende Weise darstellen. Ein Methandiphosphonsäuretetraalkylester wird in Form seines Alkalisalzes (hergestellt z.B. durch Reaktion mit Natriumhydrid in Toluol oder Dimethylformamid) mit einem -Halogenalkancarbonsäurebenzylester z.B. dem Bromessigsäurebenzylester umgesetzt. Nach erfolgter Reaktion wird die Benzylschutzgruppe durch katalytische Hydrierung in Gegenwart von Edelmetallkatalysatoren wie z.B. Palladium auf Kohle abgespalten. Man erhält in diesem Fall Verbindungen der allgemeinen Formel V, in der "A" gleich Wasserstoff bedeutet. Falls "A" die Hydroxyigruppe darstellt, erhält man die gewünschte Verbindung in Form ihres Benzylesters analog dem im Houben-Weyl 12/1, 453, 481 beschriebenen Verfahren durch Umsetzung eines Alkandicarbonsäuremonobenzylesterchlorids z.B. Bernsteinsäurebenzylesterchlorid mit einem Trialkylphosphit zu einem Acylphosphonat, das anschließend unter schwach basischer Katalyse mit einem Dialkylphosphit zur Reaktion gebracht wird. Anschließend werden auch hier die entstandenen Benzylester, wie oben beschrieben, zu den freien Carbonsäuren umgesetzt.

Bei Verfahren III entsprechen die Verbindungen der allgemeinen Formel VI für Z = NH den Verbindungen der allgemeinen Formel IV und werden, wie dort beschrieben, hergestellt. Falls Z = Schwefel darstellt, handelt es sich im Fall Y = Valenzstrich um ω-Mercaptocarbonsäuren, (zur Darstellung s. z.B. J. Am. Chem. Soc. 73, 4464 und 100, 7086; Org. Synth. 34, 42) die man in der Regel am Schwefelatom geschützt in Form ihrer Säurehalogenide, vor allem Säurechloride zur Reaktion bringt. Als Schutzgruppe verwendet man vorzugsweise eine Acyl-, insbesondere die Acetyl- bzw. Benzoylgruppe (Lit beispielsweise: Chem. Ber. 74, 1751, DOS 2.703.828). Die Umsetzung der Carboxyl- in die Säurechloridgruppe erfolgt nach literaturbekannten Methoden, beispielsweise durch Umsetzung mit Phosphorpentachlorid. Falls Z = Schwefel darstellt und Y = NH bedeutet, erhält man die Verbindungen der allgemeinen Formel VI durch Umsetzung eines am Schwefelatom durch z.B. Acetyl- oder Benzoyl geschützten Thioamins mit einer Verbindung der allgemeinen Formel XII. Die verwendeten Thioamine sind käuflich oder in der Literatur beschrieben.

Verbindungen der allgemeinen Formel VII lassen sich z.B. durch Umsetzung von Dihalogenalkanen wie z.B. Chlorbromethan, -propan oder -butan mit dem Natriumsalz eines Methandiphosphonsäuretetraalkylesters darstellen. In diesem Fall erhält man Verbindungen der allgemeinen Formel VII mit A = Wasserstoff. Vorzugsweise setzt man bei den Verfahren III jedoch einen Methylenmethandiphosphonsäuretetraalkylester (J. Org. Chem. 51, 3488) oder einen Oxiran-1.1-diphosphonsäuretetraalkylester (DOS 2.117.876) ein.

Die Säurehalogenide der allgemeinen Formel VIII erhält man nach allgemein üblichen Verfahren durch Reaktion der entsprechenden Carbonsäure mit einem Halogenierungsmittel wie z.B. Thionylchlorid oder Phosphorpentachlorid. Die hierzu eingesetzten Carbonsäuren lassen sich für den Fall, daß Z = C(O)-NH ist, aus den Aminodiphosphonsäurederivaten der allgemeinen Formel III durch Umsetzung mit Dicarbonsäureanhydriden herstellen. Man kann statt dessen auch Dicarbonsäureesterchloride einsetzen und nach erfolgter Reaktion den Carbonsäurester nach üblichen Methoden zur freien Carbonsäure verseifen oder im Falle des Benzylesters in Gegenwart von Edelmetallkatalysatoren zur freien Carbonsäure hydrieren. Falls Z die NH-C(O)-Gruppe bedeutet, gelangen Aminocarbonsäuren mit einem Diphosphonsäurederivat der allgemeinen Formel V zur Reaktion. Diese Aminocarbonsäuren sind in der Regel käuflich zu erwerben oder literaturbekannt. Im Falle, daß Z die NH-Gruppe oder Schwefel bedeutet, werden die Verbindungen durch Umsetzung der oben erwähnten Aminocarbonsäuren oder Thioalkancarbonsäuren mit einem Diphosphonsäurederivat der allgemeinen Formel VII erhalten.

Die bei Verfahren V a verwendeten Isocyanato- bzw. Isothiocyanato-Verbindungen der allgemeinen Formel X lassen sich analog dem bei Verfahren I angegebenen Methoden aus den entsprechenden Aminen der allgemeinen Formel XI herstellen. Diese erhält man, für den Fall, daß Z die Gruppe -C(O)-NH darstellt, nach allgemein in der Peptidchemie gängigen Verfahren durch Umsetzung einer am Stickstoff geschützten Aminosäure, deren Carboxylgruppe in einer reaktiven Form vorliegt. Die verwendeten Schutzgruppen sind z.B. im Houben-Weyl Band 15/1 ausführlich beschrieben. Bevorzugt werden als Schutzgruppe Aralkyloxycarbonylgruppen, insbesondere die Benzyloxycarbonylaber auch die Tritylgruppe, deren Abspaltung durch katalytische Hydrierung in Gegenwart von Edelmetallkatalysatoren wie z.B. Palladium auf Kohle vorgenommen werden kann. Als aktivierte Carboxylgruppe finden die bei Verfahren I und II angegebenen Reste Verwendung. Falls Z die NH-C(O)-Gruppe bedeutet, erhält man die Verbindung der allgemeinen Formel XI durch Umsetzung eines Diamins mit einem Diphosphonsäurederivat der allgemeinen Formel V. Die verwendeten Diamine sind in der Regel käuflich oder literaturbekannt. Für den Fall, daß Z die NH-Gruppe oder Schwefel bedeutet, erhält man die gewünschten Verbindungen durch Umsetzung eines Aminothiols oder eines an einem Stickstoffatom geschützten Diamins mit einem Diphosphonsäurederivat der allgemeinen Formel VII. Die verwendeten Aminothiole bzw. Diamine sind in der Regel käuflich oder literaturbekannt. Als Schutzgruppe findet vorzugsweise eine Aralkyloxycarbonylgruppe, insbesondere die Benzyloxycarbonyl- oder auch die Tritylgruppe Verwendung, die anschließend durch katalytische Hydrierung in Gegenwart von Edelmetallkatalysatoren wie Palladium auf Kohle abhydriert werden können.

Die bei Verfahren V b verwendeten 17-β-Östradiolderivate der allgemeinen Formel XII sind für den Fall, daß W = Chlor bedeutet bekannt (EP 341.961). Falls W die Gruppe X-M bedeutet, handelt es sich vorzugsweise um Kohlensäureester, die aus den Verbindungen der allgemeinen Formel IX durch Reaktion mit Chlorameisensäureestern erhältlich sind.

Die freien Diphosphonsäuren der allgemeinen Formel I können durch Erhitzen mit Orthoameisensäurealkylestern in die entsprechenden Tetraaalkylester überführt werden und zu Diestern oder wieder zu den freien Tetrasäuren verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung der Ester zu freien Diphosphonsäuren geschieht in der Regel durch Behandlung mit Trimethylsilylhalogenid, vorzugsweise dem Bromid oder Jodid.

Als pharmakologisch verträgliche Salze werden vor allem Mono- bzw. Dialkali- oder Ammoniumsalze verwendet, die in üblicher Weise z. B. durch Titration der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl-, Triethyl- oder Cyclohexylamin herstellt werden.

Die Salze werden in der Regel durch Behandeln mit Wasser/Methanol oder Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 0.1-100 mg/Mensch, vorzugsweise bei 1-10 mg/Mensch und können auf einmal oder mehrere Male verteilt eingenommen werden.

Das nachfolgende Beispiel zeigt eine der Verfahrensvarianten, die zur Synthese der erfindungsgemäßen Verbindungen verwendet werden können. Es soll jedoch nicht eine Einschränkung des Erfindungsgegenstandes darstellen. Die Struktur dieser Verbindung ist durch ¹H- und ³¹P-NMR-Spektroskopie gesichert, die Reinheit wurde mittels ³¹-P-NMR-Spektroskopie, Dünnschichtelektrophorese (Cellulose, Oxalat-Puffer von pH = 4.0) und mittels C, H, N, P, Na-Analyse bestimmt.

### Beispiel 1

### Bernsteinsäure-[17-(estra-1,3,5(10)-trien-3,17-diol(17β))]ester-N-succinimid (1)

Bernsteinsäure-[17-(estra-1,3,5(10)-trien-3,17-diol(17β))]ester (siehe J. Biol. Chem. 253, 8221 (1978)) (2.8 g, 7.5 mmol) gelöst in 20 ml Tetrahydrofuran wird nach Zugabe von N-Hydroxisuccinimid (950 mg, 8.25 mmol), N,N'-Dicyclohexylcarbodiimid (1.7 g, 8.25 mmol) und 4-Dimethylaminopyridin (50 mg, 0.4 mmol) 12 Stunden bei Raumtemperatur gerührt. Die Reaktionsmischung wird abfiltriert, das Filtrat im Vakuum eingeengt und der Rückstand durch Chromatographie über Kieselgel mit Toluol/Aceton (5:1) gereinigt.
Ausbeute 3 g (88 % d.Th.), Schmp. 209-210°C.

### Bernsteinsäure-[17-(estra-1,3,5(10)-trien-3,17-diol(17β))]ester-N-[(3,3-diphosphonsäuretetraethylester)propyl]amid (2)

Bernsteinsäure-(17-(estra-1,3,5(10)-trien-3,17-diol(17β))]ester-N-succinimid (500 mg, 1.1 mmol) wird mit 3-Aminopropan-1,1-diphosphonsäure-tetraethylester (s. EP 098 567) (320 mg, 1.2 mmol) in 20 ml Methylenchlorid 18 Stunden bei Raumtemperatur gerührt. Die Reaktionsmischung wird mit Wasser (2x20 ml) und 2 N HCl (2x10 ml) extrahiert, die organische Phase über wasserfreiem Magnesiumsulfat getrocknet, im Vakuum eingeengt und zur Reinigung über Kieselgel mit Toluol/Aceton (5:1) chromatographiert.
Ausbeute 630 mg (86 % d.Th.).

### Bernsteinsäure-[17-(estra-1,3,5(10)-trien-3,17-diol(17β))]ester-N-[(3,3-diphosphono)propyl]amid (3)

Zu Bernsteinsäure-[17-(estra-1,3,5(10)-trien-3,17-diol(17β))]ester-N-[(3,3-diphosphonsäuretetraethylester)propyl]amid (500 mg, 0.73 mmol) in 10 ml Methylenchlorid wird bei 0°C Trimethylsilylbromid (560 mg, 3.65 mmol) zugegeben. Nach 12 Stunden bei Raumtemperatur wird mit gesättigter Natriumcarbonat-Lösung hydrolysiert, mit Methylenchlorid extrahiert und die organische Phase im Vakuum eingeengt.
Ausbeute: 350 mg (84 % d.Th.); Fp > 300 °C.

### Beispiel 2

### Cyclohexen-4,5-dicarbonsäure-4-[17-(estra-1,3(10)-trien-3,17-diol(17β))]ester (4)

Östradiol (1,08 g, 4 mmol), gelöst in 15 ml abs. Pyridin, wird mit cis-4-Cyclohexen-1,2-dicarbonsäureanhyrid (3 g, 20 mmol) 4 Std. bei 140°C am Rückfluß gekocht. Nach dem Abkühlen wird Wasser (60 ml), Methanol (40 ml) und Kaliumcarbonat (4 g) zugegeben und die Reaktionsmischung 2 Std. bei R. T. gerührt. Dann wird auf 6N HCl gegeben und abgesaugt. Der Rückstand wird mit Toluol/Aceton (3:1) über Kieselgel chromatographiert.
Ausb.: 1.2 g (72 % d. Th.); R_{f} (Toluol/Aceton (3:1)) : 0.25.

### Cyclohexen-4,5-dicarbonsäure-4-[17-(estra-1,3(10)-trien-3,17-diol(17β))]ester-5-N-succinimid (5)

4 (850 mg, 20 mmol) wird in abs. THF gelöst und mit N-Hydroxysuccinimid (280 mg, 24 mmol), N,N'-Dicyclohexylcarbodiimid (420 mg, 20 mmol) und 4-Dimethylaminopyridin (100 mg, 10 mmol) 12 Std. bei R.T. gerührt. Die Reaktionsmischung wird abfiltriert, im Vakuum eingeengt und der Rückstand mit Toluol/Aceton (3:1) über Kieselgel chromatographiert.
Ausb.: 920 mg (88 % d. Th.); R_{f} (Toluol/Aceton (3:1)) : 0.48.

### Cyclohexen-4,5-dicarbonsäure-4-[17-(estra-1,3,5(10)-trien-3,17-diol(17β))]-ester-5-[N-(3,3-diphosphonsäuretetraethylester)propyl]amid (6)

5 (700 mg, 13 mmol) wird mit 3-Aminopropan-1,1-diphosphonsäuretetraethylester (490 mg, 15 mmol) in abs. Methylenchlorid (20 ml) analog 2 umgesetzt. Der Rückstand wird mit Toluol/Aceton (1:1) über Kieselgel chromatographiert.
Ausb.: 730 mg (76 % d. Th.); R_{f} (Essigester/Aceton (1:3)):0.56

### Cyclohexen-4,5-dicarbonsäure-4-[17-(estra-1,3,5(10)-trien-3,17-diol(17β))]-ester-5-[N-(3,3-diphosphono)propyl]amid (7)

Zu einer Lösung von 6 (1 g, 1.5 mmol) in 10 ml abs. Acetonitril wird bei -20° C Trimethylsilyljodid (1.5 ml, 11 mmol) zugetropft. Die Mischung wird auf 0° C erwärmt und 2 Std. bei R.T. nachgerührt. Das Lösungsmittel wird abgezogen, der Rückstand wird mit Wasser (20 ml) und Methylenchlorid (20 ml) versetzt und noch weitere 30 min. bei 0° C gerührt. Die wäßrige Phase wird 3x mit Methylenchlorid gewaschen und eingeengt. Der Rückstand wird mit aus Wasser/Ethanol umkristallisiert
Ausb.: 610 mg (75 % d. Th.); Fp. > 300° C

### Beispiel 3

### Cyclohexan-1,2-dicarbonsäure-1-[17-(estra-1,3,5(10)-trien-3,17-diol(17β))]ester (8)

Östradiol (1.35, 5 mmol) wird analog 4 mit Cyclohexan-1,2-dicarbonsäureanhydrid umgesetzt und aufgearbeitet. Der Rückstand mit Toluol/Aceton (5:1) über Kieselgel chromatographiert.
Ausb.: 1.5 g (70 % d. Th.); R_{f} (Toluol/Aceton (5:1)):0.30

### Cyclohexan-1,2-dicarbonsäure-1-[17-estra-1,3,5(10)-trien-3,17-diol(17β))]ester-2-N-succinimid (9)

8 (800 mg, 20 mmol) wird analog 5 umgesetzt. Der Rückstand wird mit Toluol/Aceton (5:1) über Kieselgel chromatographiert.
Ausb.: 890 mg (85 % d. Th.); R_{f} (Toluol/Aceton (5:1)):0.43.

### Cyclohexan-4,5-dicarbonsäure-4-[17-(estra-1,3,5(10)-trien-3,17-diol(17β))]-ester-5-[N-(3,3-diphosphonsäuretetraethylester)-propyl]amid (10)

9 (2.6 g, 50 mmol) wird analog 2 mit 3-Aminopropan-1,1-diphosphonsäuretetraethylester umgesetzt. Der Rückstand wird mit Toluol/Aceton (2:3) über Kieselgel chromatographiert.
Ausb.: 2.8 g (76 % d. Th.); R_{f} (Essigester/Aceton (2:1)):0.50

### Cyclohexan-4,5-dicarbonsäure-4-[17-(estra-1,3,5(10-trien-3,17-diol(17β))]-ester-5-[N-(3,3-diphosphono)propyl]amid (11)

10 (1.0 g, 1.5 mmol) wird analog 7 umgesetzt. Die Wasserphase wird 3 x mit Methylenchlorid und 3 x mit Diethylether gewaschen und eingeengt. Der Rückstand wird aus Wasser/Ethanol umkristallisiert.
Ausb.: 610 mg (65 % d. Th.); Fp. > 300° C

### Beispiel 4

### Phthalsäure-1-[17-(estra-1,3,5(10)-trien-3,17-diol(17β))]-ester (12)

Östradiol (2.7 g, 10 mmol) werden analog 4 mit Phthalsäureanhydrid (7 g, 50 mmol) umgesetzt und aufgearbeitet.
Ausb.: 3.5 g (84 % d. Th.); Fp. 234-235° C

### Phthalsäure-[17-(estra-1,3,5(10)-trien-3,17-diol(17β))]ester-N-succinimid (13)

12 (1.7 g, 40 mmol) wird analog 5 umgesetzt. Der Rückstand wird mit Toluol/Aceton (7:1) über Kieselgel chromatographiert.
Ausb.: 625 mg (65 % d. Th.); R_{f} (Toluol/Aceton (7:1):0.61

### Phthalsäure-[17-(estra-1,3,5(10)-trien-3,17-diol(17β))]ester-[N-(3,3-diphosphonsäuretetraethylester-propyl]amid (14)

13 (700 mg, 13 mmol) wird analog 2 mit 3-Aminopropan-1,1-diphosphonsäuretetraethylester (500 mg, 15 mmol) umgesetzt. Der Rückstand wird mit Toluol/Aceton (1:2) über Kieselgel chromatographiert.
Ausb.: 670 mg (69 % d. Th.); R_{f} (Toluol/Aceton (1:2)):0.49

### Phthalsäure-[17-(estra-1,3,5,(10)-trien-3,17-diol(17β))]ester-[N-(3,3-diphosphono)propyl]amid (15)

14 (220 mg, 3 mmol) werden analog 7 umgesetzt. Der Rückstand wird aus Wasser/Ethanol umkristallisiert.
Ausb.: 130 mg (70 % d. Th.); Fp. > 300° C

### Beispiel 5

### 3-0-Benzyl-estra-1,3,5(10)-trien-3,17diol(17β) (16)

Zu einer Suspension aus Natriumhydrid (0.5 g, 23.3 mmol) in abs. DMF (10 ml) wird bei -5° C Östradiol (4.3 g, 15.8 mmol), gelöst in abs. DMF (10 ml), zugetropft. Nach 1 Std. bei -5° C wird Benzylbromid (2.8 ml, 23.3 mmol) zugetropft und 1 Std. bei R.T. nachgerührt. Die Reaktionsmischung wird über Kieselgel abfiltriert und im Hochvakuum eingeengt, mit 10 % Zitronensäure versetzt (150 ml) und 3 x mit Diethylether extrahiert. Die Etherphase wird mit gesättigter Natriumhydrogencarbonatlösung neutral gewaschen, über Magnesiumsulfat getrocknet und eingeengt. Der Rückstand wird mit Heptan kristallisiert.
Ausb.: 4.6 g (80 % d. Th.); Fp. 98-100° C; R_{f} (Heptan/Essigester (2:1)):0.37

### Chlorameisensäure-17-(3-0-benzyl-estra-1,3,5(10)-trien-3,17-diol(17β))ester (17)

16 (3.6 g, 10 mmol) wird in abs. Dioxan (90 ml) gelöst, mit Aktivkohle (150 mg) und Diphosgen (1.2 ml, 11 mmol) versetzt und 2 Std. am Rückfluß gekocht. Die Reaktionsmischung wird abgekühlt, filtriert und eingeengt.
Ausb.: 3 g (70 % d. Th.); R_{f} (Heptan/Essigester (2:1)):0.80

### 3-0-Benzyl-estra-1,3,5(10)-trien-3,17-diol(17β)-17-[N-(4,4-diphosphono-4-hydroxy)butyl]carbon (Monokaliumsalz) (18)

4-Amino-1,1-diphosphono-butanol (1.5 g, 6 mmol) wird in Wasser gelöst und mit 1 N KOH auf pH 10.5 eingestellt. Dann wird 17 (3 g, 7 mmol), gelöst in THF, zugetropft. Der pH-Wert wird durch Zugabe von 1 N KOH zwischen 10 und 11 gehalten. Es wird 12 Std. nachgerührt und eingeengt. Der Rückstand wird in Wasser aufgenommen über eine Membran (0.2 µm) filtriert und eingeengt. Der Rückstand wird aus Wasser/Ethanol umkristallisiert.
Ausb.: 3.35 g (71 % d. Th.); Fp. > 300° C

### 3-Estra-1,3,5-(10)-trien-3,17-diol(17β)-17-[N-(4,4-dihosphono-4-hydroxy)butyl]carbamat (Monokaliumsalz) (19)

18 (1.5 g, 2.4 mmol) wird in Dioxan/Wasser (60 ml) gelöst und unter Zusatz von Kaliumcarbonat (240 mg, 2.4 mmol) bei 3.5 bar mit Pd/C 10 % als Katalysator unter H₂ hydriert. Die Mischung wird filtriert und eingeengt. Der Rückstand wird in Wasser aufgenommen über eine Membran (0.1 µm) filtriert und mit Methylenchlorid extrahiert. Die wäßrige Phase wird eingeengt und der Rückstand aus Wasser/Ethanol umkristallisiert.
Ausb.: 940 mg (65 % d. Th.); Fp. > 300° C

### Beispiel 6

### 3-0-Benzyl-estra-1,3,5(10)trien-3,17diol(17β)-17-[N-(3,3-diphosphono)propyl]carbamat (Monokaliumsalz) (20)

3-Amino-1,1-diphosphonsäure (850 mg, 4 mmol) wird in Wasser gelöst und mit 1 N KOH auf pH 11 gestellt. Dann wird 17 (1.9 g, 4.5 mmol) gelöst in THF zugetropft. Die weitere Umsetzung und Aufarbeitung erfolgt analog 18.
Ausb.: 1.6 g (63 % d. Th.); Fp. > 300° C

### 3-Estra-1,3,5(10)trien-3,17-diol(17β)-17-[N-(3,3-diphosphono)propyl]carbamat (Monokaliumsalz) (21)

20 (650 mg, 1 mmol) wird in Dioxan/Wasser unter Zusatz von Kaliumcarbonat analog 19 hydriert und aufgearbeitet.
Ausb.: 400 mg (72 % d. Th.); Fp. > 300° C
Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonsäuren, sowie deren Natriumsalze, Methyl- bzw. Ethylester:
Methylmalonsäure-[17-(estra-1,3,5(10)-trien-3,17-diol(17β))]-ester-N-(3,3-diphosphono-propyl)-amid
Pentylmalonsäure-[17-(estra-1,3,5(10)-trien-3,17-diol(17β))]-ester-N-(3,3-diphosphono-propyl)-amid
Allylmalonsäure-[17-(estra-1,3,5(10)-trien-3,17-diol(17β))]-ester-N-(3,3-diphosphono-propyl)-amid
Phenylmalonsäure-[17-(estra-1,3,5(10)-trien-3,17-diol(17β))]-ester-N-(3,3-diphosphono-propyl)-amid
Bernsteinsäure-[17-(estra-1,3,5(10)-trien-3,17-diol(17β))]-ester-N-(3,3-diphosphono-propyl)-amid
2-Methylbernsteinsäure-[17-(estra-1,3,5(10)-trien-3,17-diol(17β))]-ester-N-(3,3-diphosphono-propyl)-amid
2,2-Dimethylbernsteinsäure-[17-(estra-1,3,5(10)-trien-3,17-diol(17β))]-ester-N-(3,3-diphosphono-propyl)-amid
Cyclohexan-1,2-dicarbonsäure-[17-(estra-1,3,5(10)-trien-3,17-diol(17β))]-ester-N-(3,3-diphosphono-propyl)-amid
Cyclohexen-4,5-dicarbonsäure-4-[17-(estra-1,3,5(10)-trien-3,17-diol(17β))]-ester-N-(3,3-diphosphono-propyl)-amid
Phthalsäure-1-[17-(estra-1,3,5(10)-trien-3,17-diol(17β))]-ester-N-(3,3-diphosphono-propyl)-amid
Pyrazin-2,3-dicarbonsäure-2-[17-(estra-1,3,5(10)-trien-3,17-diol(17β))]-ester-N-(3,3-diphosphono-propyl)-amid
Glutarsäure-1-[17-(estra-1,3,5(10)-trien-3,17-diol(17β))]-ester-N-(3,3-diphosphono-propyl)-amid
3-Methylglutarsäure-1-[17-(estra-1,3,5(10)-trien-3,17-diol(17β))]-ester-N-(3,3-diphosphono-propyl)-amid
3-Phenylglutarsäure-1-[17-(estra-1,3,5(10)-trien-3,17-diol(17β))]-ester-N-(3,3-diphosphono-propyl)-amid
3,3-Dimethylglutarsäure-1-[17-(estra-1,3,5(10)-trien-3,17-diol(17β))]-ester-N-(3,3-diphosphono-propyl)-amid
3,3-Tetramethylenglutarsäure-1-[17-(estra-1,3,5(10)-trien-3,17-diol(17β))]-ester-N-(3,3-diphosphono-propyl)-amid
Cyclohexan-1,3-dicarbonsäure-1-[17-(estra-1,3,5(10)-trien-3,17-diol(17β))]-ester-N-(3,3-diphosphono-propyl)-amid
Decandisäure-1-[17-(estra-1,3,5(10)-trien-3,17-diol(17β))]-ester-N-(3,3-diphosphono-propyl)-amid
Malonsäure-[17-(estra-1,3,5(10)-trien-3,17-diol(17β))]-ester-N-(3,3-diphosphonopropyl)-amid
2-tert.-Butylmalonsäure-[17-(estra-1,3,5(10)-trien-3,17-diol(17β))]-ester-N-(5,5-diphosphono-5-hydroxy-valeryl)-amid
Cyclohexylmalonsäure-1-[17-(estra-1,3,5(10)-trien-3,17-diol(17β))]-ester-N-(4,4-diphosphono-4-hydroxy-4-butyl)-amid
Benzylmalonsäure-1-[17-(estra-1,3,5(10)-trien-3,17-diol(17β))]-ester-N-(7,7-diphosphono-7-hydroxy-heptyl)-amid
2,2-Dimethylmalonsäure-1-[17-(estra-1,3,5(10)-trien-3,17-diol(17β))]-ester-N-(6,6-diphosphono-6-hydroxy-hexyl)-amid
2,2-Dipentylmalonsäure-1-[17-(estra-1,3,5(10)-trien-3,17-diol(17β))]-ester-N-(3,3-diphosphono-3-hydroxy-propyl)-amid
2,2-Dimethylmalonsäure-1-[17-(estra-1,3,5(10)-trien-3,17-diol(17β))]-ester-N-(4,4-diphosphono-4-hydroxy-butyl)-amid
2,2-Diallylmalonsäure-1-[17-(estra-1,3,5(10)-trien-3,17-diol(17β))]-ester-N-(7,7-diphosphono-7-hydroxy-heptyl)-amid
Cyclopropan-1,1-dicarbonsäure-1-[17-(estra-1,3,5(10)-trien-3,17-diol(17β))]-ester-N-(4,4-diphosphono-4-hydroxy-butyl)-amid
Cyclobutan-1,1-dicarbonsäure-1-[17-(estra-1,3,5(10)-trien-3,17-diol(17β))]-ester-N-(3,3-diphosphono-3-hydroxy-propyl)-amid
Cyclopentan-1,1-dicarbonsäure-1-[17-(estra-1,3,5(10)-trien-3,17-diol(17β))]-ester-N-(5,5-diphosphono-5-hydroxy-pentyl)-amid
Cyclohexan-1,1-dicarbonsäure-1-[17-(estra-1,3,5(10)-trien-3,17-diol(17β))]-ester-N-(2,2-diphosphono-2-hydroxy-ethyl)-amid
2-Hexyl-bernsteinsäure-1-[17-(estra-1,3,5(10)-trien-3,17-diol(17β))]-ester-N-(2,2-diphosphono-2-hydroxy-ethyl)-amid
2,3-Dimethyl-bernsteinsäure-[17-(estra-1,3,5(10)-trien-3,17-diol(17β))]-ester-N-(2,2-diphosphono-2-hydroxy-ethyl-amid
3,3-Dimethylbernsteinsäure-1-[17-(estra-1,3,5(10)-trien-3,17-diol(17β))]-ester-N-(7,7-diphosphono-7-hydroxy-heptyl)-amid
Cyclopropan-1,2-dicarbonsäure-1-[17-(estra-1,3,5(10)-trien-3,17-diol(17β))]-ester-N-(2,2-diphosphono-2-hydroxy-ethyl)-amid
Cyclobutan-1,2-dicarbonsäure-1-[17-(estra-1,3,5(10)-trien-3,17-diol(17β))]-ester-N-(4,4-diphosphono-4-hydroxy-butyl)-amid
Pyridin-2,3-dicarbonsäure-2-[17-(estra-1,3,5(10)-trien-3,17-diol(17β))]-ester-N-(3,3-diphosphono-propyl)-amid
Pyridin-5,6-dicarbonsäure-5-[17-(estra-1,3,5(10)-trien-3,17-diol(17β))]-ester-N-(3,3-diphosphono-propyl)-amid
Pyridin-3,4-dicarbonsäure-3-[17-(estra-1,3,5(10)-trien-3,17-diol(17β))]-ester-N-(3,3-diphosphono-3-hydroxy-propyl)-amid
Pyridin-4,5-dicarbonsäure-4-[17-(estra-1,3,5(10)-trien-3,17-diol(17β))]-ester-N-(4,4-diphosphono-4-hydroxy-butyl)-amid
2-Methylvaleriansäure-1-[17-(estra-1,3,5(10)-trien-3,17-diol(17β))]-ester-N-(3,3-diphosphono-3-hydroxy-propyl)-amid
4-Methylvaleriansäure-1-[17-(estra-1,3,5(10)-trien-3,17-diol(17β))]-ester-N-(3,3-diphosphono-3-hydroxy-propyl)-amid
2,4-Dimethylvaleriansäure-1-[17-(estra-1,3,5(10)-trien-3,17-diol(17β))]-ester-N-(5,5-diphosphono-5-hydroxy-pentyl)-amid
2,2-Dimethylvaleriansäure-1-[17-(estra-1,3,5(10)-trien-3,17-diol(17β))]-ester-N-(7,7-diphosphono-7-hydroxy-heptyl)-amid
4,4-Dimethylvaleriansäure-1-[17-(estra-1,3,5(10)-trien-3,17-diol(17β))]-ester-N-(3,3-diphosphono-3-hydroxy-propyl)-amid
Pentamethylenglutarsäure-1-[17-(estra-1,3,5(10)-trien-3,17-diol(17β))]-ester-N-(4,4-diphosphono-4-hdyroxy-butyl)-amid
Cyclopentan-1,3-dicarbonsäure-1-[17-(estra-1,3,5(10)-trien-3,17-diol(17β))]-ester-N-(2,2-diphosphono-2-hydroxy-ethyl)-amid
Hexandicarbonsäure-[17-(estra-1,3,5(10)-trien-3,17-diol(17β))]-ester-N-(5,5-diphosphono-5-hydroxy-pentyl)-amid
Heptandicarbonsäure-[17-(estra-1,3,5(10)-trien-3,17-diol(17β))]-ester-N-(6,6-diphosphono-6-hydroxy-hexyl)-amid
N-(5,5-Diphosphono-5-hydroxy-valeroyl)-glycin-[17-(estra-1,3,5(10)-trien-3,17-diol(17β))]-ester
N-(4,4-Diphosphono-buttersäure)-L-alanin-[17-(estra-1,3,5(10)-trien-3,17-diol(17β))]-ester
N-(4,4-Diphosphono-buttersäure)-L-phenylalanin-[17-(estra-1,3,5(10)-trien-3,17-diol(17β))]-ester
N-(5,5-Diphosphono-5-hydroxy-valeroyl)-2-methyl-L-alanin-[17-(estra-1,3,5(10)-trien-3,17-diol(17β))]-ester
N-(3,3-Diphosphono-propionyl)-3-aminopropionsäure-[17-(estra-1,3,5(10)-trien-3,17-diol(17β))]-ester
N-(3,3-Diphosphono-propionyl)-2-aminocyclohexancarbonsäure-[17-(estra-1,3,5(10)-trien-3,17-diol(17β))]-ester
N-(3,3-Diphosphono-propionyl)-4-aminocyclohexancarbonsäure-[17-(estra-1,3,5(10)-trien-3,17-diol(17β))]-ester
N-(3,3,-Diphosphono-propionyl)-4-aminobenzoesäure-[17-(estra-1,3,5(10)-trien-3,17-diol(17β))]-ester
N-(5,5-Diphosphono-5-hydroxy-valeroyl)-6-aminohexansäure-[17-(estra-1,3,5(10)-trien-3,17-diol(17β))]-ester
N-(3,3-Diphosphono-propionyl)-9-aminononansäure-[17-(estra-1,3,5(10)-trien-3,17-diol(17β))]-ester
N-(5,5-Diphosphono-5-hydroxy-valeroyl)-L-leucin-[17-(estra-1,3,5(10)-trien-3,17-diol(17β))]-ester
N-(5,5-Diphosphono-5-hydroxy-valeroyl)-2-amino-2,2-tetramethylen-essigsäure-[17-(estra-1,3,5(10)-trien-3,17-diol(17β))]-ester
N-(3,3-Diphosphono-propionyl)-2-amino-2,2-pentamethylenessigsäure-[17-(estra-1,3,5(10)-trien-3,17-diol(17β))]-ester
N-(5,5-Diphosphono-5-hydroxy-valeroyl)-2-aminobenzoesäure-[17-(estra-1,3,5(10)-trien-3,17-diol(17β))]-ester
N-(5,5-Diphosphono-5-hydroxy-valeroyl)-4-aminobuttersäure-[17-(estra-1,3,5(10)-trien-3,17-diol(17β))]-ester
N-(3,3-Diphosphono-propionyl)-4-amino-2,2-dimethylbuttersäure-[17-(estra-1,3,5(10)-trien-3,17-diol(17β))]-ester
N-(5,5-Diphosphono-5-hydroxy-valeroyl)-3-aminocyclohexancarbonsäure-[17-(estra-1,3,5(10)-trien-3,17-diol(17β))]-ester
N-(5,5-Diphosphono-5-hydroxy-valeroyl)-3-aminobenzoesäure-[17-(estra-1,3,5(10)-trien-3,17-diol(17β))]-ester
N-(3,3-Diphosphono-propionyl)-5-aminovaleriansäure-[17-(estra-1,3,5(10)-trien-3,17-diol(17β))]-ester
N-(3,3-Diphosphono-propionyl)-4-methylamino-cyclohexancarbonsäure-[17-(estra-1,3,5(10)-trien-3,17-diol(17β))]-ester
N-(Estra-1,3,5(10)-trien-3,17-diol(17β)-17-carbonyl)-2-aminoessigsäure-N-(3,3-diphosphono-propyl)-amid
N-(Estra-1,3,5(10)-trien-3,17-diol(17β)-17-carbonyl)-3-aminopropionsäure-N-(3,3-diphosphono-propyl)-amid
N-(Estra-1,3,5(10)-trien-3,17-diol(17β)-17-carbonyl)-L-alanin-N-(5,5-diphosphono-5-hydroxy-valeryl)-amid
N-(Estra-1,3,5(10)-trien-3,17-diol(17β)-17-carbonyl)-4-aminobuttersäure-N-(5,5-diphosphono-5-hydroxy-valeryl)-amid
N-(Estra-1,3,5(10)-trien-3,17-diol(17β)-17-carbonyl)-5-aminovaleriansäure-N-(3,3-diphosphono-propyl)-amid
N-(Estra-1,3,5(10)-trien-3,17-diol(17β)-17-carbonyl)-4-aminocyclohexancarbonsäure-N-(3,3-diphosphono-propyl)-amid
N-(Estra-1,3,5(10)-trien-3,17-diol(17β)-17-carbonyl)-4-aminomethyl-cyclohexancarbonsäure-N-(5,5-diphosphono-5-hydroxyvaleryl)-amid
N-(Estra-1,3,5(10)-trien-3,17-diol(17β)-17-carbonyl)-4-aminobenzoesäure-N-(3,3-diphosphono-3-propyl)-amid
N-(Estra-1,3,5(10)-trien-3,17-diol(17β)-17-thia)-4-aminopropionsäure-N-(3,3-diphosphono-propyl)-amid
N-(Estra-1,3,5(10)-trien-3,17-diol(17β)-17-thia)-3-aminocyclohexansäure-N-(3,3-diphosphono-propyl)-amid
N-(Estra-1,3,5(10)-trien-3,17-diol(17β)-17-thia)-L-alanin-N-(5,5-diphosphono-5-hydroxy-valeryl)-amid
N-(Estra-1,3,5(10)-trien-3,17-diol(17β)-17-thia)-2-aminoessigsäure-N-(5,5-diphosphono-5-hydroxy-valeryl)-amid
N-(Estra-1,3,5(10)-trien-3,17-diol(17β)-17-thia)-4-aminobenzoesäure-N-(3,3-diphosphono-propyl)-amid
N-(Estra-1,3,5(10)-trien-3,17-diol(17β)-17-thia)-2-aminobenzoesäure-N-(5,5-diphosphono-5-hydroxy-valeryl)-amid
N-(Estra-1,3,5(10)-trien-3,17-diol(17β)-17-thia)-4-aminobuttersäure-N-(3,3-diphosphono-propyl)-amid
N-(Estra-1,3,5(10)-trien-3,17-diol(17β)-17-thia)-3-aminocrotonsäure-N-(3,3-diphosphono-propyl)-amid
N-(Estra-1,3,5(10)-trien-3,17-diol(17β)-17-carbonyl)-N'-(3,3,-diphosphono-propionyl)-ethylendiamin
N-(Estra-1,3,5(10)-trien-3,17-diol(17β)-17-carbonyl)-N'-(3,3-diphosphono-propionyl)-1,2-diaminocyclohexan
N-(Estra-1,3,5(10)-trien-3,17-diol(17β)-17-carbonyl)-N'-(5,5-diphosphono-5-hydroxy-valeroyl)-1,1-dimethylethylendiamin
N-(Estra-1,3,5(10)-trien-3,17-diol(17β)-17-carbonyl)-N'-(5,5-diphosphono-5-hydroxy-valeroyl)-1,3-diaminopropan
N-(Estra-1,3,5(10)-trien-3,17-diol(17β)-17-carbonyl)-N'-(3,3-diphosphono-propionyl)-1,3-diamino-2,2-dimethylpropan
N-(Estra-1,3,5(10)-trien-3,17-diol(17β)-17-carbonyl)-N'-(3,3-diphosphono-propienyl)-1,4-diaminobutan
N-(Estra-1,3,5(10)-trien-3,17-diol(17β)-17-carbonyl)-N'-(3,3-diphosphono-propionyl)-1,4-diaminocyclohexan
N-(Estra-1,3,5(10)-trien-3,17-diol(17β)-17-carbonyl)-N'-(5,5-diphosphono-valeroyl)-1,4-diamino-1,1,4,4-tetramethylbutan
N-(Estra-1,3,5(10)-trien-3,17-diol(17β)-17-carbonyl)-N'-(3,3-diphosphono-propionyl)-1,6-diaminohexan
N-(Estra-1,3,5(10)-trien-3,17-diol(17β)-17-carbonyl)-N'-(5,5-diphosphono-5-hydroxy-valeroyl)-1,8-diaminooctan
N-(2',2'-Diphosphono-ethyl)-L-leucin-[17-(estra-1,3,5(10)-trien-3,17-diol(17β))]-ester
N-(2',2'-Diphosphono-ethyl)-4-aminobuttersäure-[17-(estra-1,3,5(10)-trien-3,17-diol(17β))]-ester
N-(2',2'-Diphosphono-ethyl)-glycin-[17-(estra-1,3,5(10)-trien-3,17-diol(17β))]-ester
N-(2',2'-Diphosphono-2-hydroxy-ethyl)-L-alanin-[17-(estra-1,3,5(10)-trien-3,17-diol(17β))]-ester
N-(2',2'-Diphosphono-ethyl)-L-phenylalanin-[17-(estra-1,3,5(10)-trien-3,17-diol(17β))]-ester
N-(2',2'-Diphosphono-2'-hydroxy-ethyl)-2-methyl-alanin-[17-(estra-1,3,5(10)-trien-3,17-diol(17β))]-ester
N-(2',2'-Diphosphono-2'-hydroxy-ethyl)-2,2-tetramethylenglycin-[17-(estra-1,3,5(10)-trien-3,17-diol(17β))]-ester
N-2',2'-Diphosphono-ethyl)-2,2-pentamethylen-glycin-[17-(estra-1,3,5(10)-trien-3,17-diol(17β))]-ester
N-(2',2'-Diphosphono-2'-hydroxy-ethyl)-3-aminopropionsäure-[17-(estra-1,3,5(10)-trien-3,17-diol(17β))]-ester
N-(2',2'-Diphosphono-ethyl)-2-aminocyclohexancarbonsäure-[17-(estra-1,3,5(10)-trien-3,17-diol(17β))]-ester
N-(2',2'-Diphosphono-2'-hydroxy-ethyl)-2-aminobenzoesäure- [17-(estra-1,3,5(10)-trien-3,17-diol(17β))]-ester
N-(2',2'-Diphosphono-2'-hydroxy-ethyl)-4-aminocyclohexancarbonsäure-[17-(estra-1,3,5(10)-trien-3,17-diol(17β))]-ester
N-(2',2'-Diphosphono-ethyl)-4-aminobenzoesäure-[17-(estra-1,3,5(10)-trien-3,17-diol(17β))]-ester
N-(2',2'-Diphosphono-2'-hydroxy-ethyl)-4-methylaminocyclohexancarbonsäure-[17-(estra-1,3,5(10)-trien-3,17-diol(17β))]-ester
N-(2',2'-Diphosphono-ethyl)-4-amino-2,2-dimethylbuttersäure-[17-(estra-1,3,5(10)-trien-3,17-diol(17β))]-ester
N-(2',2'-Diphosphono-2'-hydroxy-ethyl)-3-amino-2,2-dimethylpropionsäure-[17-(estra-1,3,5(10)-trien-3,17-diol(17β))]-ester
N-(2',2'-Diphosphono-ethyl)-3-aminocyclohexancarbonsäure-[17-(estra-1,3,5(10)-trien-3,17-diol(17β))]-ester
N-(2',2'-Diphosphono-2'-hydroxy-ethyl)-3-aminobenzoesäure-[17-(estra-1,3,5(10)-trien-3,17-diol(17β))]-ester
N-(2',2'-Diphosphono-2'-hydroxy-ethyl)-6-aminohexansäure-[17-(estra-1,3,5(10)-trien-3,17-diol(17β))]-ester
N-(2',2'-Diphosphono-ethyl)-9-aminononansäure-[17-(estra-1,3,5(10)-trien-3,17-diol(17β))]-ester
3-(2',2'-Diphosphono-2'-hydroxy-ethylmercapto)-propionsäure-[17-(estra-1,3,5(10)-trien-3,17-diol(17β))]-ester
2-(2',2'-Diphosphono-2'-hydroxy-ethylmercapto)-benzoesäure-[17-(estra-1,3,5(10)-trien-3,17-diol(17β))]-ester
4-(2',2'-Diphosphono-ethylmercapto)-buttersäure-[17-(estra-1,3,5(10)-trien-3,17-diol(17β))]-ester
5-(2',2'-Diphosphono-2'-hydroxy-ethylmercapto)-valeriansäure-[17-(estra-1,3,5(10)-trien-3,17-diol(17β))]-ester
9-(2',2'-Diphosphono-2'-hydroxy-ethylmercapto)-nonansäure-[17-(estra-1,3,5(10)-trien-3,17-diol(17β))]-ester
3-(2',2'-Diphosphono-ethylmercapto)-3-methyl-propionsäure-[17-(estra-1,3,5(10)-trien-3,17-diol(17β))]-ester
3-(2',2'-Diphosphono-ethylmercapto)-2-methyl-propionsäure-[17-(estra-1,3,5(10)-trien-3,17-diol(17β))]-ester
2-(2',2'-Diphosphono-ethylmercapto)-essigsäure-[17-(estra-1,3,5(10)-trien-3,17-diol(17β))]-ester
2-(2',2'-Diphosphono-ethylmercapto)-2-methyl-essigsäure-[17-(estra-1,3,5(10)-trien-3,17-diol(17β))]-ester
4-(2',2'-Diphosphono-2'-hydroxy-ethylmercapto)-benzoesäure-[17-(estra-1,3,5(10)-trien-3,17-diol(17β))]-ester
4-[2'-(2'',2''-Diphosphono-ethylmercapto)-ethyl]-benzoesäure-[17-(estra-1,3,5(10)-trien-3,17-diol(17β))]-ester
N-(Estra-1,3,5(10)-trien-3,17-diol(17β)-17-carbonyl)-N'-(2,2-diphosphono-ethyl)-1,3-diaminopropan
N-(Estra-1,3,5(10)-trien-3,17-diol(17β)-17-carbonyl)-N'-(2,2-diphosphono-ethyl)-1,3-diamino-2,2-dimethylpropan
N-(Estra-1,3,5(10)-trien-3,17-diol(17β)-17-carbonyl)-N'-(2,2-diphosphono-ethyl)-ethylendiamin
N-(Estra-1,3,5(10)-trien-3,17-diol(17β)-17-carbonyl)-N'-(2,2-diphosphono-2-hydroxy-ethyl)-1,1-dimethylethylendiamin
N-(Estra-1,3,5(10)-trien-3,17-diol(17β)-17-carbonyl)-N'-(2,2-diphosphono-2-hydroxy-ethyl)-1,2-diaminocyclohexan
N-(Estra-1,3,5(10)-trien-3,17-diol(17β)-17-carbonyl)-N'-(2,2-diphosphono-2-hydroxy-ethyl)-1,4-diaminobutan
N-(Estra-1,3,5(10)-trien-3,17-diol(17β)-17-carbonyl)-N'-(2,2-diphosphono-ethyl)-1,4-diaminocyclohexan
N-(Estra-1,3,5(10)-trien-3,17-diol(17β)-17-carbonyl)-N'-(2,2-diphosphono-ethyl)-1,4-diamino-2,2,4,4-tetramethyl-butan
N-(Estra-1,3,5(10)-trien-3,17-diol(17β)-17-carbonyl)-N'-(2,2-diphosphono-ethyl)-1,6-diaminohexan
N-(Estra-1,3,5(10)-trien-3,17-diol(17β)-17-carbonyl)-N'-(2,2-diphosphono-2-hydroxy-ethyl)-1,8-diaminooctan
2-(2',2'-Diphosphono-ethylmercapto)-N-(estra-1,3,5(10)-trien-3,17-diol(17β)-17-carbonyl)-ethylamin
2-(2',2'-Diphosphono-ethylmercapto)-N-(estra-1,3,5(10)-trien-3,17-diol(17β)-17-carbonyl)-cyclohexylamin
2-(2',2'-Diphosphono-ethylmercapto)-N-(estra-1,3,5(10)-trien-3,17-diol(17β)-17-carbonyl)-butylamin
Estra-1,3,5(10)-trien-3,17diol(17β)-17-[N-(3,3-diphosphonopropyl)]-carbamat
Estra-1,3,5(10)-trien-3,17diol(17β)-17-[N-(4,4-diphosphono-4-hydroxy)butyl]-carbamat

## Patentansprüche

1. Verbindungen der allgemeinen Formel I 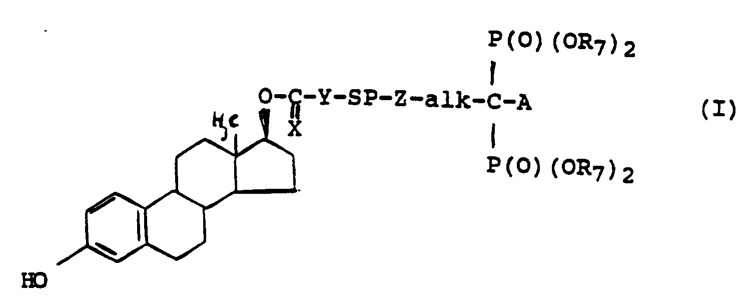 in der
Sp 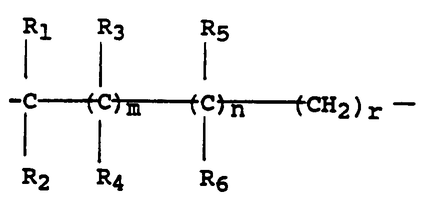
R₁-R₆ = jeweils unabhängig voneinander Wasserstoff, Phenyl, Cyclohexyl, niederes geradkettiges oder verzweigtes, Alkyl, Alkenyl oder Alkinyl mit 1-5 bzw. 2-5 Kohlenstoffatomen, das gegebenenfalls durch Phenyl substituiert ist, wobei R₁ und R₂ zusammen mit dem Kohlenstoffatom, an das sie gebunden sind einen gesättigten alicyclischen 3-6 Ring bilden können, bzw. R₁ und R₅ zusammen mit dem/den sie verbindenden Kohlenstoffatom(en) einen gegebenenfalls durch 1 oder 2 Stickstoffatome unterbrochenen gesättigten, teilgesättigten oder ungesättigten 3-6-Ring bilden können,
m, n und r unabhängig voneinander Werte von 0-3 einnehmen können, wobei m + n + r höchstens den Wert 7 einnehmen darf,
und
X = 0 oder S
Y = Valenzstrich oder NH
Z = Valenzstrich, 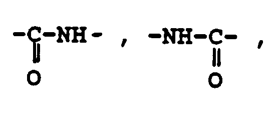 NH oder S,
A = Wasserstoff, Hydroxy oder gegebenenfalls durch niederes Alkyl substituiertes Amino,
alk = eine geradkettige oder verzweigte Alkylenkette mit 1-6 Kohlenstoffatomen,
R₇ = Wasserstoff oder niederes Alkyl
bedeuten, sowie deren pharmakologisch unbedenklichen Salze.

2. Verfahren zur Herstellung von Verbindungen der allgemeinen Formel I 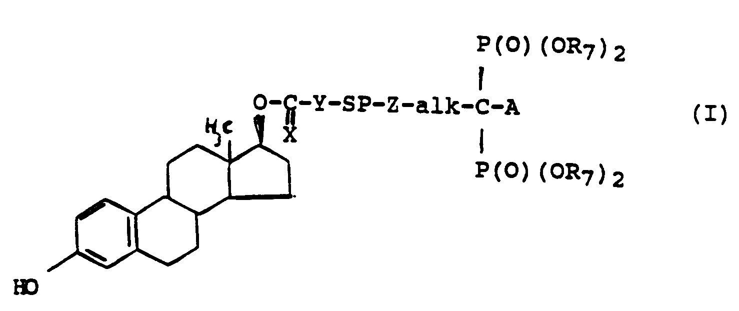 in der
Sp 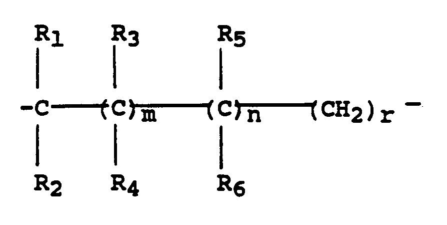
R₁-R₆ = jeweils unabhängig voneinander Wasserstoff, Phenyl, Cyclohexyl, niederes geradkettiges oder verzweigtes, Alkyl, Alkenyl oder Alkinyl mit 1-5 bzw. 2-5 Kohlenstoffatomen, das gegebenenfalls durch Phenyl substituiert ist, wobei R₁ und R₂ zusammen mit dem Kohlenstoffatom, an das sie gebunden sind einen gesättigten alicyclischen 3-6 Ring bilden können, bzw. R₁ und R₅ zusammen mit dem/den sie verbindenden Kohlenstoffatom(en) einen gegebenenfalls durch 1 oder 2 Stickstoffatome unterbrochenen gesättigten, teilgesättigten oder ungesättigten 3-6-Ring bilden können,
m, n und r unabhängig voneinander Werte von 0-3 einnehmen können, wobei m + n + r höchstens den Wert 7 einnehmen darf,
und
X = 0 oder S
Y = Valenzstrich oder NH
Z = Valenzstrich, 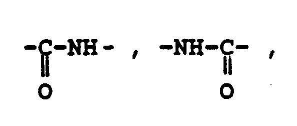 NH oder S,
A = Wasserstoff, Hydroxy oder gegebenenfalls durch niederes Alkyl substituiertes Amino,
alk = eine geradkettige oder verzweigte Alkylenkette mit 1-6 Kohlenstoffatomen,
R₇ = Wasserstoff oder niederes Alkyl
bedeuten, sowie deren pharmakologisch unbedenklichen Salze,
dadurch gekennzeichnet, daß man in an sich bekannter Weise
I für den Fall, daß Z die 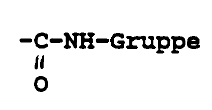 bedeutet,
ein Carbonsäurederivat der allgemeinen Formel II 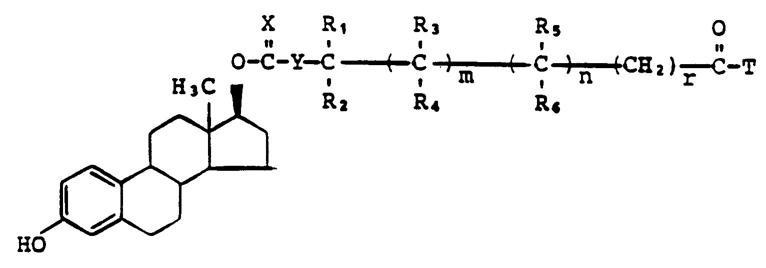 in der X, Y, R₁-R₆, m, n und r die oben angegebenen Bedeutungen haben und T die Hydroxigruppe oder eine Aktivierungsgruppe darstellt, mit einem Diphosphonsäurederivat der allgemeinen Formel III 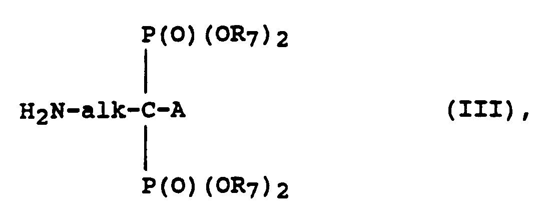 in der alk, R₇ und A die oben angegebenen Bedeutungen haben, umsetzt, oder
II) für den Fall, daß Z die Gruppe 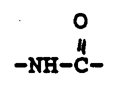 bedeutet, eine Aminoverbindung der allgemeinen Formel IV 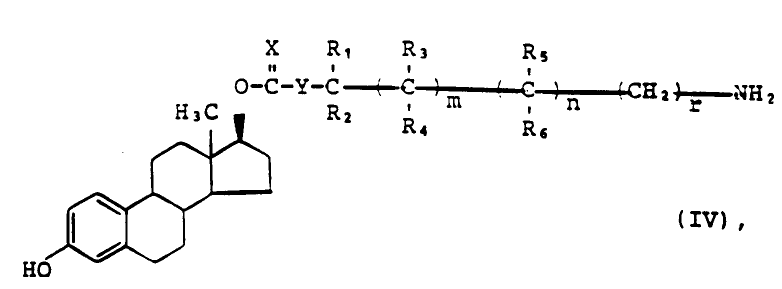 in der X, Y, R₁-R₆, m, n und r die oben genannten Bedeutungen haben, mit einem Diphosphonsäurederivat der allgemeinen Formel V 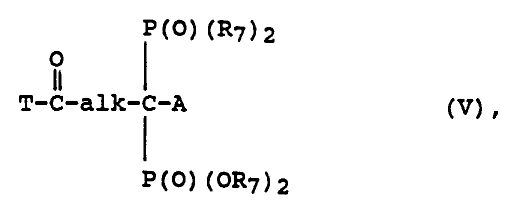 in der alk, A und R₇ die oben angegebenen Bedeutungen haben und T die Hydroxi- oder eine Aktivierungsgruppe darstellt, umsetzt, oder
III für den Fall, daß Z = NH oder S bedeutet, eine Verbindung der allgemeinen Formel VI 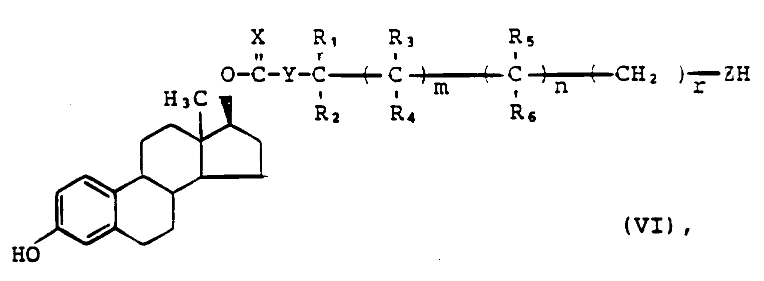 in der X, Y, R₁-R₆, m, n und r die oben genannten Bedeutungen haben und Z = NH oder S ist, mit einem Diphosphonsäurederivat der allgemeinen Formel VII 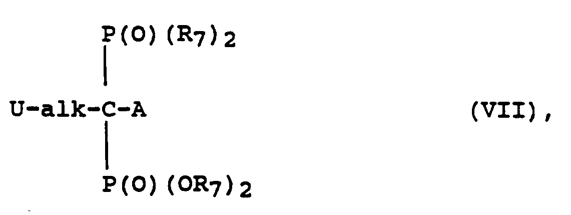 wobei alk, A und R₇ die oben angegebenen Bedeutungen haben und U einen reaktiven Rest darstellt, wobei für den Fall, daß alk eine C₁-Gruppe bedeutet, U und A zusammen auch einen Valenzstrich oder ein Sauerstoffatom darstellen können, umsetzt, oder
IV) für den Fall, daß X = Sauerstoff und Y = Valenzstrich darstellen, eine Verbindung der allgemeinen Formel VIII 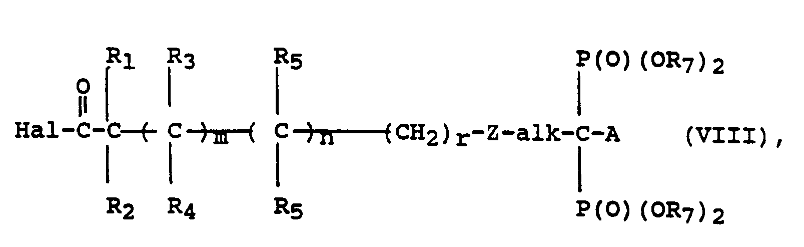 in der R₁-R₇, Z, alk, A, m, n und r die oben angegebenen Bedeutungen haben und Hal = Chlor oder Brom ist, mit einem 17-β-Östradiolderivat der allgemeinen Formel IX 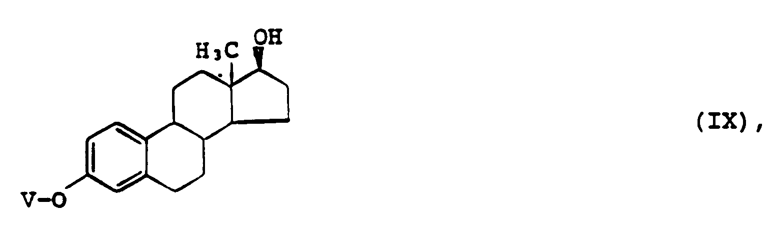 in der V eine Schutzgruppe darstellt, umsetzt, oder
V) für den Fall, daß Y = NH ist,
a) eine Verbindung der allgemeinen Formel X 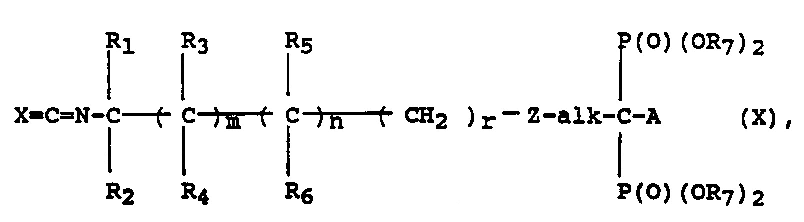 in der X, R₁-R₇, Z, alk, A, m, n und r die oben angegebenen Bedeutungen haben, mit einem 17-β-Östradiolderivat der allgemeinen Formel IX 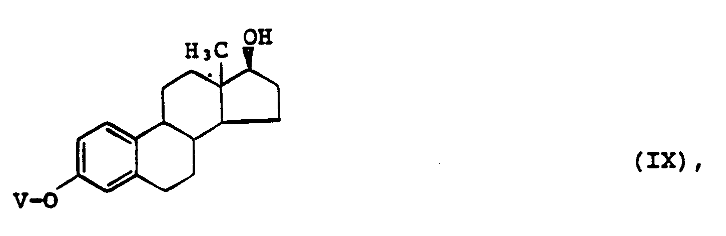 in der V eine Schutzgruppe darstellt, umsetzt, oder
b) eine Aminoverbindung der allgemeinen Formel XI 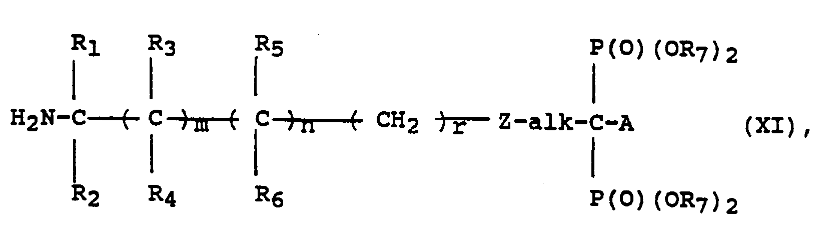 in der R₁-R₇, Z, alk, A, m, n und r die oben angegebenen Bedeutungen haben, mit einem 17-β-Östradiolderivat der allgemeinen Formel XII 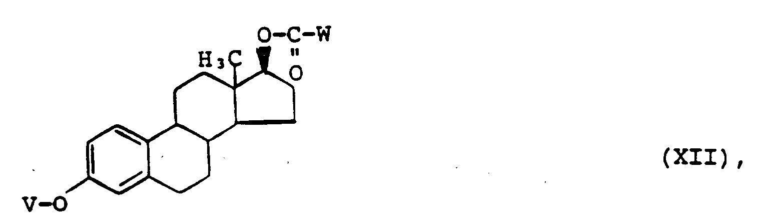 in der V eine Schutzgruppe darstellt und W = Chlor oder die Gruppe -X-M sein soll, wobei M niederes Alkyl wie Methyl oder Ethyl oder einen gegebenenfalls substituierten Phenylring darstellt und X die oben angegegebene Bedeutung hat, umsetzt, und anschließend gewünschtenfalls die C=O-Gruppe in eine C=S-Gruppe überführt oder
VI) Verbindungen der allgemeinen Formel I, in der X = Sauerstoff darstellt, nachträglich in Verbindungen der allgemeinen Formel I mit X = Schwefel umwandelt,
die gegebenenfalls vorhandenen Schutzgruppen abspaltet und gewünschtenfalls die entstandenen Tetraester zu Diestern oder Säuren der allgemeinen Formel I verseift
und die erhaltenen Verbindungen gewünschtenfalls in pharmakologisch unbedenkliche Salze überführt.

3. Arzneimittel, enthaltend eine Verbindung der Formel I gemäß Anspruch 1 nebst üblichen Träger- und Hilfsstoffen.

4. Verwendung von Verbindungen der Formel I gemäß Anspruch 1 zur Herstellung von Arzneimitteln zur Behandlung der Osteoporose.

## Claims

1. Compounds of the general formula I 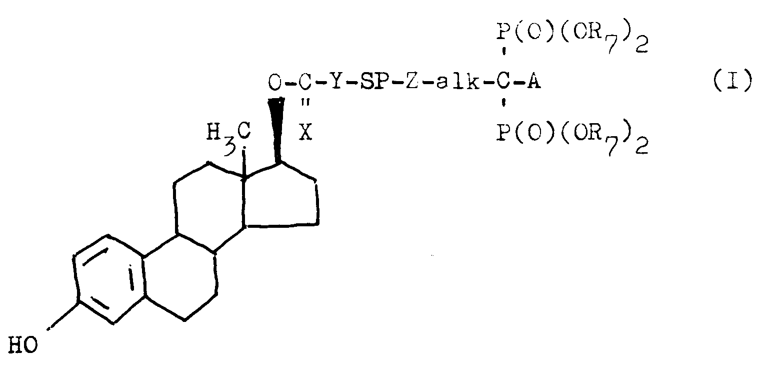 in which Sp signifies 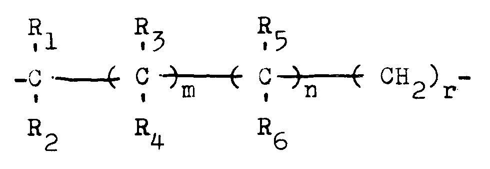
R₁ - R₆ = in each case independently of one another, hydrogen, phenyl, cyclohexyl, lower straight-chained or branched alkyl, alkenyl or alkynyl with 1 - 5 and 2 - 5 carbon atoms, respectively, which is possibly substituted by phenyl, whereby R₁ and R₂, together with the carbon atoms to which they are attached, can form a saturated alicyclic 3 - 6 ring or R₁ and R₅, together with the carbon atom(s) connecting them can form a saturated, partly saturated or unsaturated 3 - 6 ring possibly interrupted by 1 or 2 nitrogen atoms.
m, n and r, independently of one another, can assume values of 0 - 3, whereby m + n + r may assume the value of at most 7,
and
X = 0 or S,
Y = valency bond or NH
Z = valency bond, 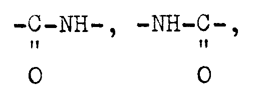 NH or S
A = hydrogen, hydroxyl or amino possibly substituted by lower alkyl,
alk = a straight-chained or branched alkylene chain with 1 - 6 carbon atoms,
R₇ = hydrogen or lower alkyl,
as well as their pharmacologically acceptable salts.

2. Process for the preparation of compounds of the general formula I 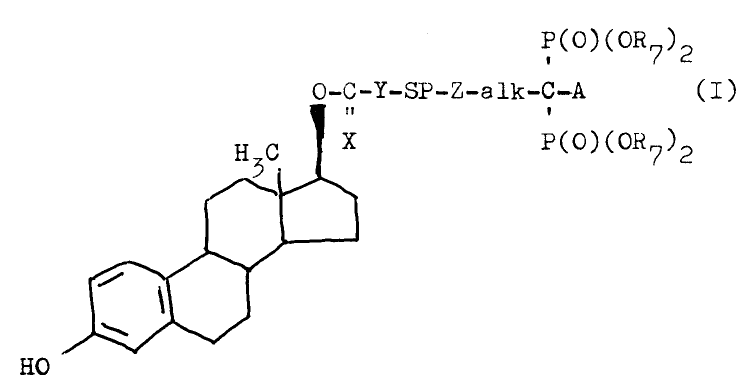 in which Sp signifies 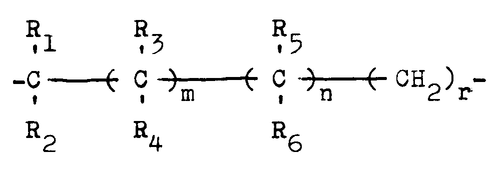
R₁-R₆ = in each case independently of one another, hydrogen, phenyl, cyclohexyl, lower straight-chained or branched alkyl, alkenyl or alkynyl with 1 - 5 and 2 - 5 carbon atoms, respectively, which is possibly substituted by phenyl, whereby R₁ and R₂, together with the carbon atom to which they are attached, can form a saturated alicyclic 3 - 6 ring or R₁ and R₂, together with the carbon atom(s) connecting them, can form a saturated, partly saturated or unsaturated 3 - 6 ring possibly interrupted by 1 or 2 nitrogen atoms,
m, n and r, independently of one another, can assume values of 0 - 3, whereby m + n + r may assume the value of at most 7, and
A = 0 or S,
Y = valency bond or NH
Z = valency bond, 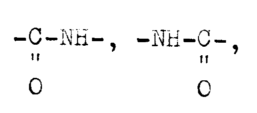 NH or S
A = hydrogen, hydroxyl or amino possibly substituted by lower alkyl,
alk = a straight-chained or branched alkylene chain with 1 - 6 carbon atoms,
R₇ = hydrogen or lower alkyl,
as well as of their pharmacologically acceptable salts, characterised in that, in per se known manner, one
I) for the case that Z signifies the 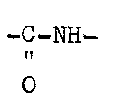 group, reacts a carboxylic acid derivative of the general formula II 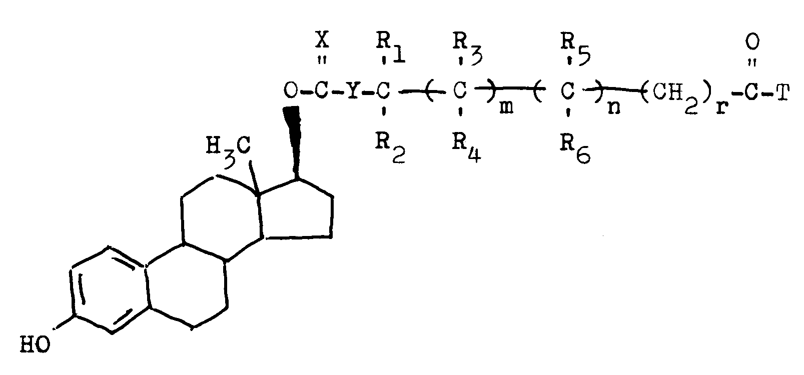 in which X, Y, R₁-R₆, m, n and r have the above-given meanings and T represents the hydroxyl group or an activating group, with a diphosphonic acid derivative of the general formula III 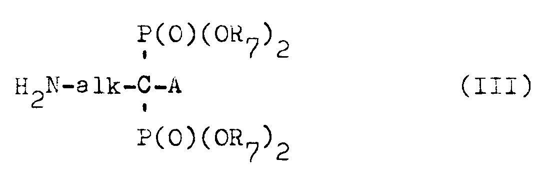 in which alk, R₇ and A have the above-given meanings, or
(II) for the case that Z signifies the group 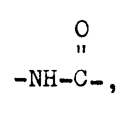 reacts an amino compound of the general formula IV 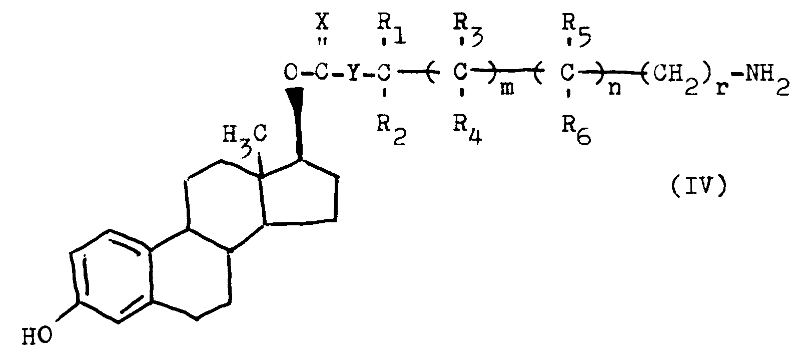 in which X, Y, R₁-R₆, m, n and r have the above-given meanings, with a diphosphonic acid derivative of the general formula V 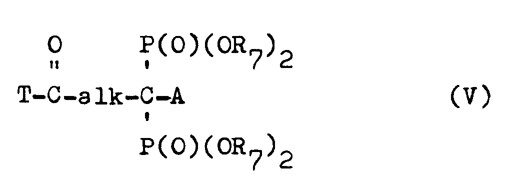 in which alk, A and R₇ have the above-given meanings and T represents the hydroxyl or an activating group, or
III) for the case that Z = NH or S, reacts a compound of the general formula VI 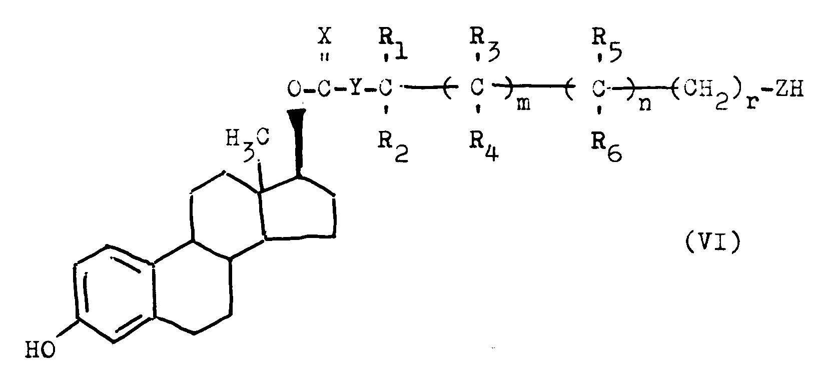 in which X, Y, R₁-R₆, m, n and r have the above-given meanings and Z = NH or S, with a diphosphonic acid derivative of the general formula VII 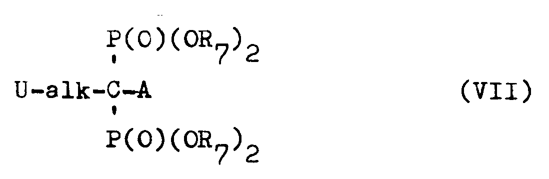 whereby alk, A and R₇ have the above-given meanings and U represents a reactive residue, whereby, for the case that alk signifies a C₁ group, U and A can together also represent a valency bond or an oxygen atom, or
IV) for the case that X = oxygen and Y = valency bond, reacts a compound of the general formula VIII 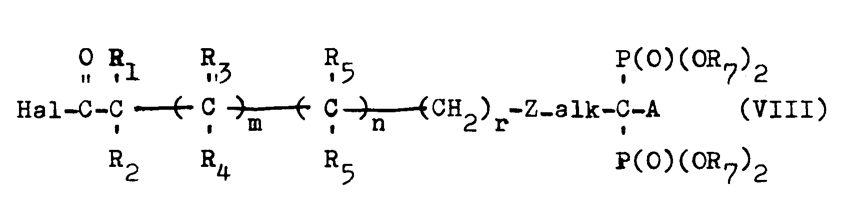 in which R₁-R₇, Z, alk, A, m, n and r have the above-given meanings and Hal = chlorine or bromine, with a 17-β-oestradiol derivative of the general formula IX 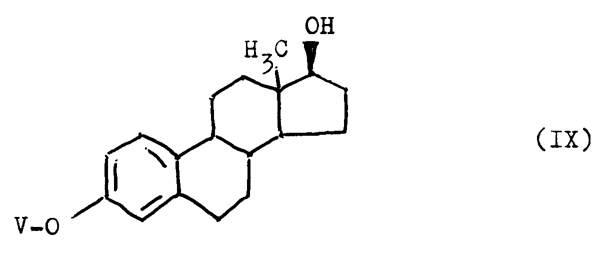 in which V represents a protective group, or
V) for the case that Y = NH,
a) reacts a compound of the general formula X 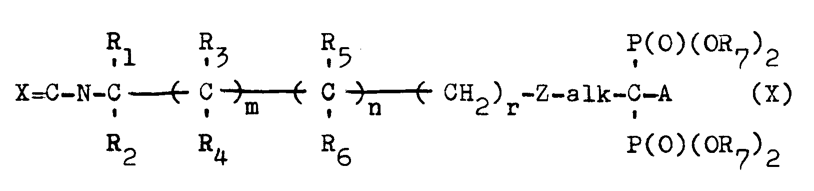 in which X, R₁-R₇, Z, alk, A, m, N and r have the above-given meanings, with a 17β-oestradiol derivative of the general formula IX 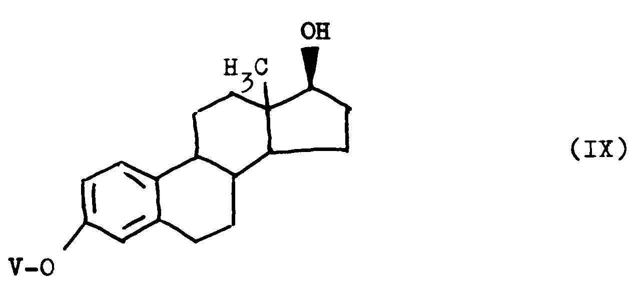 in which V represents a protective group, or
b) reacts an amino compound of the general formula XI 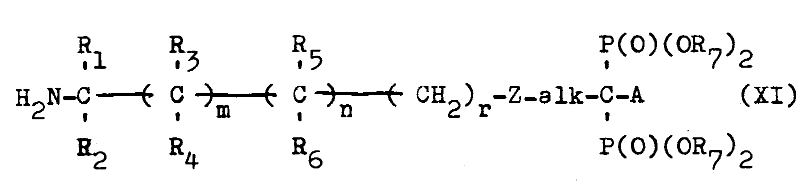 in which R₁-R₇, Z, alk, A, m, n and r have the above-given meanings, with a 17β-oestradiol derivative of the general formula XII 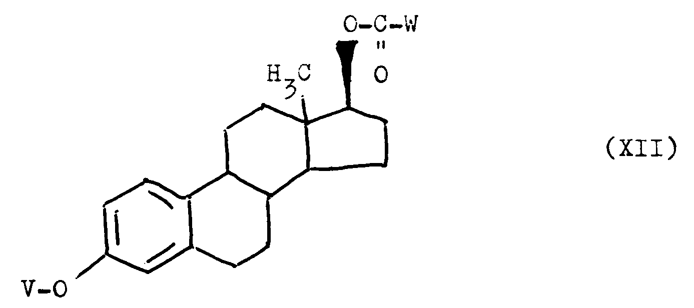 in which V represents a protective group and W = is to be chlorine or the group -X-M, whereby M represents lower alkyl, such as methyl or ethyl, or a possibly substituted phenyl ring and X has the above-given meaning, and subsequently, if desired, converts the C=O group into a C=S group, or
VI) subsequently converts compounds of the general formula I, in which X = oxygen, into compounds of the general formula I with X = sulphur, splits off the protective groups possibly present and, if desired, saponifies the resultant tetraesters to diesters or acids of the general fomula I and, if desired, converts the compounds obtained into pharmacologically acceptable salts.

3. Medicaments containing a compound of the formula I according to claim 1, besides usual carrier and adjuvant materials.

4. Use of compounds of the formula I according to claim 1 for the preparation of medicaments for the treatment of osteoporosis.

## Revendications

1. Composés de formule générale I 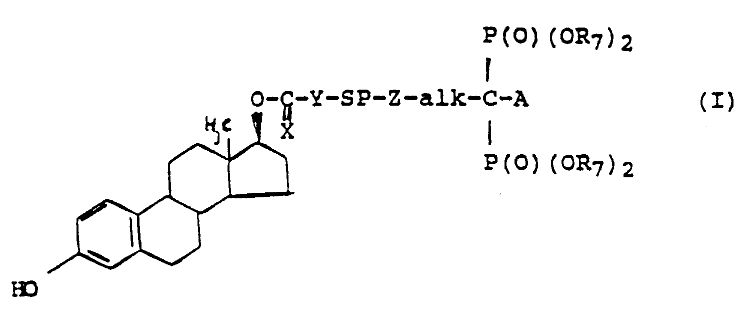 dans laquelle
Sp représente 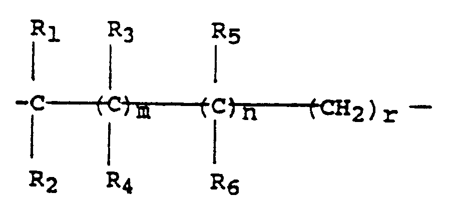
R₁ -R₆ = chacun indépendamment, un atome d'hydrogène, un groupe phényle, un groupe cyclohexyle, un groupe alkyle, alcényle ou alcinyle inférieur, à chaîne linéaire ou ramifiée, ayant 1-5 ou 2-5 atomes de carbone, qui peut être éventuellement substitué par un groupe phényle, R₁ et R₂ pouvant former, avec l'atome de carbone auquel ils sont liés, un cycle en 3-6 alicyclique, saturé, ou R₁ et R₅ pouvant former, avec le ou les atomes de carbone qui les relient, un cycle en 3-6 saturé, partiellement saturé ou insaturé, éventuellement interrompu par 1 ou 2 atomes d'azote,
m, n et r peuvent prendre, indépendamment les uns des autres, une valeur de 0-3, la somme m+n+r ne devant pas être supérieure à 7,
et
X = O ou S
Y = une liaison ou NH
Z = une liaison, 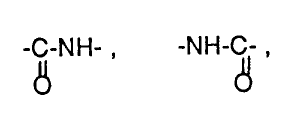 NH ou S,
A = un atome d'hydrogène, un groupe hydroxy ou un groupe amino éventuellement substitué par un groupe alkyle inférieur,
alk = une chaîne alkyle linéaire ou ramifiée, ayant 1-6 atomes de carbone,
R₇ = un atome d'hydrogène ou un groupe alkyle inférieur,
ainsi que leurs sels pharmacologiquement acceptables.

2. Procédé de préparation de composés ayant la formule générale I 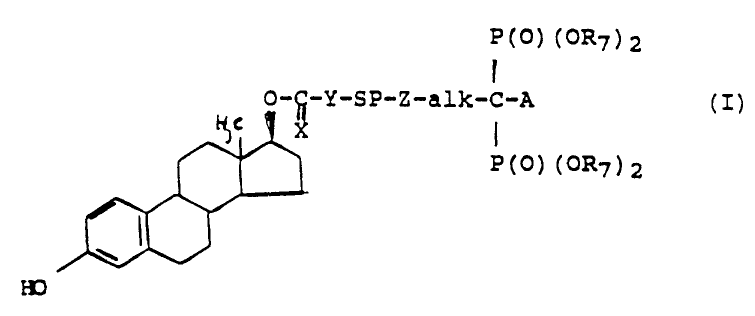 dans laquelle
Sp représente 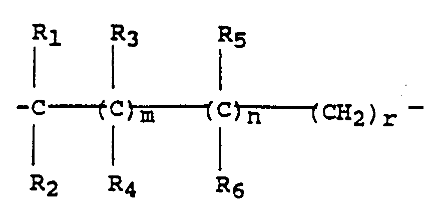
R₁-R₆ = chacun indépendamment un atome d'hydrogène, un groupe phényle, un groupe cyclohexyle, un groupe alkyle, alcényle ou alcinyle inférieur, à chaîne linéaire ou ramifiée, ayant 1-5 ou 2-5 atomes de carbone, qui peut être éventuellement substitué par un groupe phényle, R₁ et R₂ pouvant former, avec l'atome de carbone auquel ils sont liés, un cycle en 3-6 alicyclique, saturé, ou R₁ et R₅ pouvant former, avec le ou les atomes de carbone qui les relient, un cycle en 3-6 saturé, partiellement saturé ou insaturé, éventuellement interrompu par 1 ou 2 atomes d'azote,
m, n et r peuvent prendre, indépendamment les uns des autres, une valeur de 0-3, la somme m+n+r ne devant pas être supérieure à 7,
et
X = O ou S
Y = une liaison ou NH
Z = une liaison, 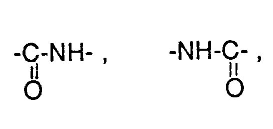 NH ou S,
A = un atome d'hydrogène, un groupe hydroxy ou un groupe amino éventuellement substitué par un groupe alkyle inférieur,
alk = une chaîne alkyle linéaire ou ramifiée, ayant 1-6 atomes de carbone,
R₇ = un atome d'hydrogène ou un groupe alkyle inférieur,
ainsi que leurs sels pharmacologiquement acceptables,
caractérisé en ce que, de manière connue en soi,
I) dans le cas où Z représente le groupe 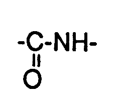 on fait réagir un dérivé d'acide carboxylique de formule générale II 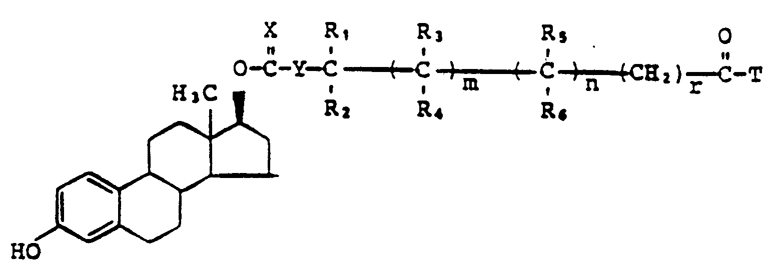 dans laquelle X, Y, R₁-R₆, m, n et r ont les significations mentionnées ci-dessus, et T représente le groupe hydroxy ou un groupe d'activation, avec un dérivé d'acide diphosphonique de formule générale III 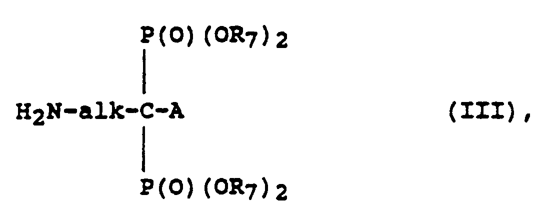 dans laquelle alk, R₇ et A ont les significations mentionnées ci-dessus, ou
II) dans le cas où Z représente le groupe 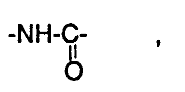 on fait réagir un composé amino de formule générale IV 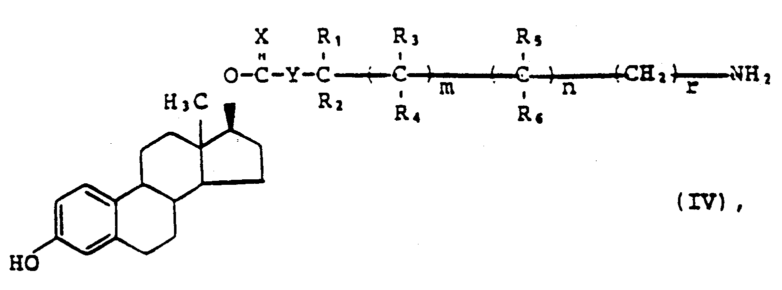 dans laquelle X, Y, R₁-R₆, m, n et r ont les significations mentionnées ci-dessus, avec un dérivé d'acide diphosphonique de formule générale V 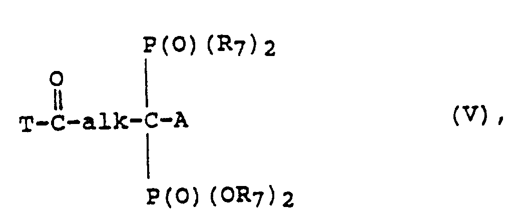 dans laquelle alk, A et R₇ ont les significations mentionnées ci-dessus, et T représente le groupe hydroxy ou un groupe d'activation, ou
III) dans le cas où Z=NH ou S, on fait réagir un composé de formule générale VI 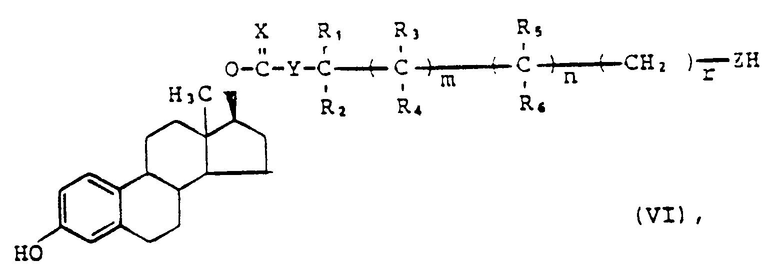 dans laquelle X, Y, R₁-R₆, m, n et r ont les significations mentionnées ci-dessus et Z=NH ou S, avec un dérivé d'acide diphosphonique de formule générale VII 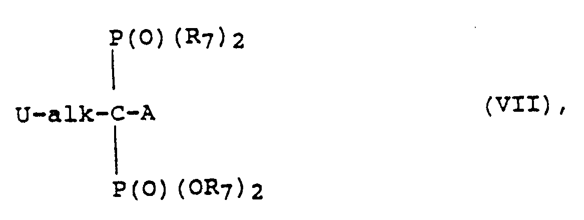 dans laquelle alk, A et R₇ ont les significations mentionnées ci-dessus et U représente un groupe réactif, U et A pouvant représenter également, dans le cas où alk signifie un groupe en C₁, une liaison ou un atome d'oxygène, ou
IV) dans le cas ou X = un oxygène et Y = une liaison, on fait réagir un composé de formule générale VIII 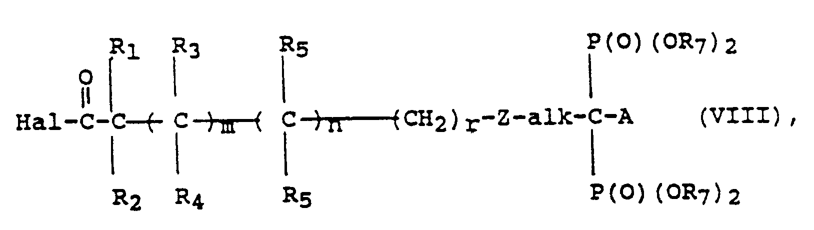 dans laquelle R₁-R₇, Z, alk, A, m, n et r ont les significations mentionnées ci-dessus et Hal = un chlore ou un brome, avec un dérivé de 17-β-oestradiol de formule générale IX 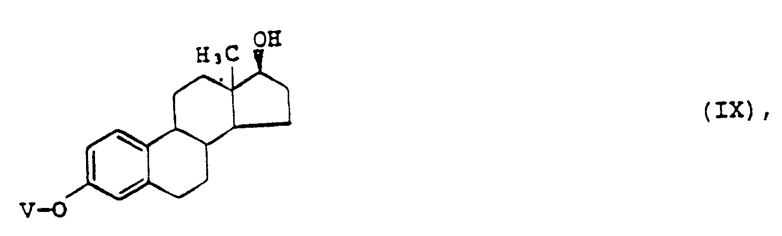 dans laquelle V représente un groupe de protection, ou
V) dans le cas où Y = NH,
a) on fait réagir un composé de formule générale X 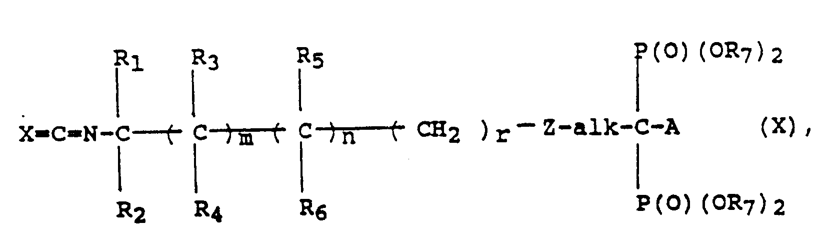 dans laquelle X, R₁-R₇, Z, alk, A, m, n et r ont les significations mentionnées ci-dessus, avec un dérivé de 17-β-oestradiol de formule générale IX 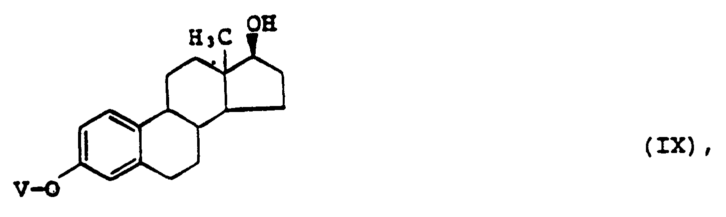 dans laquelle V représente un groupe de protection, ou
b) on fait réagir un composé amino de formule générale XI 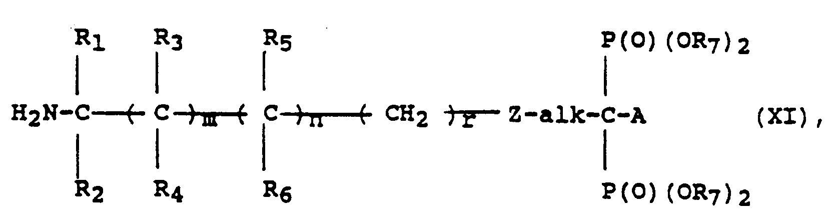 dans laquelle R₁-R₇, Z, alk, A, m, n et r ont les significations mentionnées ci-dessus, avec un dérivé de 17-β-oestradiol de formule générale XII 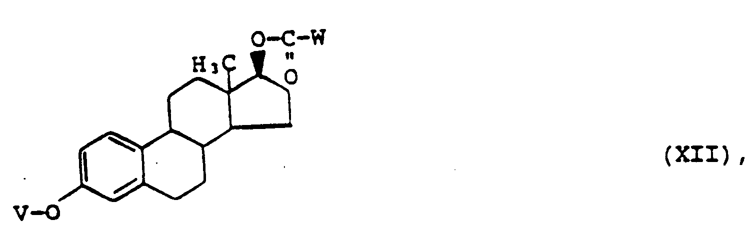 dans laquelle V représente un groupe de protection et W = un chlore ou un groupe -X-M, où M représente un groupe alkyle inférieur tel qu'un groupe méthyle ou éthyle ou un noyau phényle éventuellement substitué et X a la signification mentionnée ci-dessus, et ensuite, on transforme éventuellement le groupe C=O en un groupe C=S, ou
VI) on transforme ultérieurement les composés de formule générale I, dans lesquels X = un oxygène, en composés de formule générale I avec X = un soufre,
on sépare les groupes de protection éventuellement présents et on saponifie éventuellement le tétraester formé en diester ou en acide de formule générale I,
et on transforme éventuellement les composés obtenus en leurs sels pharmacologiquement acceptables.

3. Médicament, contenant un composé de formule I selon la revendication 1, en plus de véhicules et adjuvants usuels.

4. Utilisation de composés de formule générale I selon la revendication 1, pour la préparation de médicaments destinés au traitement de l'ostéoporose.
